# EUROPÄISCHE PATENTSCHRIFT

(11) **EP 3 019 586 B1**
(45) Veröffentlichungstag und Bekanntmachung des Hinweises auf die Patenterteilung: **21.08.2024**
(21) Anmeldenummer: 14738380.6
(22) Anmeldetag: 25.06.2014
(51) Int. Cl.: C12M 1/00, C12M 1/12

(54) **KULTIVIERUNGSGEFÄSS UND VERFAHREN ZUR KULTIVIERUNG BIOLOGISCHER ZELLEN IN HÄNGENDEN TROPFEN**
CULTURE VESSEL AND METHOD FOR CULTURING BIOLOGICAL CELLS IN HANGING DROPS
BOÎTE DE CULTURE ET PROCÉDÉ DE CULTURE DE CELLULES BIOLOGIQUES DANS DES GOUTTES SUSPENDUES

(30) Priorität: 10.07.2013 DE 102013011534
(43) Veröffentlichungstag der Anmeldung: 18.05.2016
(73) Patentinhaber: Fraunhofer-Gesellschaft zur Förderung der angewandten Forschung e.V., 80686 München (DE)
(72) Erfinder: ZIMMERMANN, Heiko, 97295 Waldbrunn (DE); FUHR, Günter R., 13187 Berlin (DE); NEUBAUER, Julia, 66386 St. Ingbert (DE)
(74) Vertreter: v. Bezold & Partner Patentanwälte - PartG mbB
(86) Internationale Anmeldenummer: PCT/EP2014/001717
(87) Internationale Veröffentlichungsnummer: WO 2015/003775

(56) Entgegenhaltungen:
- WO-A1-00/47323
- WO-A1-2008/125347
- WO-A1-2012/014047
- WO-A2-2011/094572
- DE-A1- 19 949 735

## Beschreibung

Die Erfindung betrifft ein Kultivierungsgefäß, das zur Kultivierung biologischer Zellen in hängenden Tropfen eingerichtet ist, insbesondere ein Kultivierungsgefäß, in dessen Innenraum eine Kultivierungsfläche zur Aufnahme der hängenden Tropfen vorgesehen ist. Des Weiteren betrifft die Erfindung ein Verfahren zur Kultivierung biologischer Zellen in hängenden Tropfen. Anwendungen der Erfindung sind bei der Kultivierung von biologischen Zellen, insbesondere von Stammzellen, gegeben.

Die Kultivierung (Vermehrung und/oder Differenzierung) biologischer Zellen in hängenden Tropfen ist ein allgemein verbreitetes Kultivierungsverfahren. In den hängenden Tropfen können die biologischen Zellen kultiviert werden, wobei ein Kontakt mit festen Substratoberflächen vermieden und eine geometrische Anordnung der Zellen ähnlich zur Anordnung von Zellen bei deren Vermehrung und Differenzierung in der Natur realisiert werden. Für die Bildung der hängenden Tropfen und deren Beladung mit den Zellen sind bisher die folgenden Techniken bekannt.

Bei einem typischerweise manuell ausgeführten Verfahren wird eine flache Schale mit einem ebenen Boden, wie z. B. der Deckel einer Petrischale, verwendet. Zunächst wird der Deckel so auf einer Unterlage angeordnet, dass eine innere Oberfläche des Deckels freiliegt. Tropfen einer Zellsuspension werden mit einer Pipette auf die Oberfläche aufgesetzt, und anschließend wird der Deckel durch eine Schwenkbewegung so umgedreht, dass die mit den Tropfen besetzte Oberfläche nach unten weist. Die Schwenkbewegung muss so schnell erfolgen, dass die Tropfen nicht verlaufen, sondern an ihren Plätzen bleiben und im Ergebnis frei an der nach dem Schwenken nach unten weisenden, inneren Oberfläche des Deckels hängen. Für die Kultivierung wird der Deckel zum Schutz gegen Austrocknung auf den dazugehörigen, mit einer wässrigen Lösung gefüllten Boden der Petrischale angeordnet. Diese Technik hat den Vorteil, einfach handhabbar zu sein. Nachteile ergeben sich jedoch aus der notwendigerweise schnellen und ruckartigen Schwenkbewegung des Deckels, die ein hohes Geschick des Anwenders erfordert und eine Automatisierung nur beschränkt ermöglicht. Außerdem können in den Tropfen unerwünschte Scherkräfte auftreten, die sich nachteilig auf empfindliche Zellen, insbesondere auf Stammzellen, auswirken.

Alternativ können hängende Tropfen an Zellkultur-Platten mit Löchern erzeugt werden, die an einer Unterseite der Platten von Halteringen zur Halterung der Tropfen durch Kapillarkräfte umgeben sind. Die Bildung der Tropfen und deren Beladung an einem der Halteringe erfolgt, indem mit einer Pipette durch das zugehörige Loch in der Platte die Zellsuspension zugeführt und als Tropfen am Haltering aufgehängt wird (siehe auch WO 2008/125347). Die Zellkultur-Platte begünstigt zwar eine Automatisierung des Kultivierungsverfahrens und eine Vermeidung der genannten Scherkräfte. Von Nachteil ist jedoch, dass diese Technik besondere Maßnahmen zur Vermeidung von unerwünschten Umgebungseinflüssen und von gegenseitigen Kontaminationen benachbarter Tropfen erfordert.

Aus DE 199 49 735 A1 ist bekannt, Flüssigkeitstropfen in einem Chip zu prozessieren, der einen Boden und eine Deckfläche aufweist, wobei der Abstand zwischen dem Boden und der Deckfläche mittels eines gefederten Rahmens veränderlich ist.

Die Aufgabe der Erfindung ist es, ein verbessertes Kultivierungsgefäß bereitzustellen, das zur Kultivierung biologischer Zellen in hängenden Tropfen eingerichtet ist und mit dem Nachteile herkömmlicher Techniken vermieden werden. Das Kultivierungsgefäß soll insbesondere eine schonende Bildung der hängenden Tropfen ermöglichen, einen Schutz der hängenden Tropfen bieten und/oder automatisierbar sein. Des Weiteren ist es eine Aufgabe der Erfindung, ein verbessertes Verfahren zur Kultivierung biologischer Zellen in hängenden Tropfen bereitzustellen, mit dem Nachteile herkömmlicher Techniken vermieden werden. Das Verfahren soll insbesondere die Bildung der hängenden Tropfen vereinfachen, wobei unerwünschte Scherkräfte minimiert oder ausgeschlossen werden können, und/oder die Bereitstellung vorbestimmter, reproduzierbarer Kultivierungsbedingungen ermöglichen.

Diese Aufgaben werden durch ein Kultivierungsgefäß und ein Kultivierungsverfahren unter Verwendung des Kultivierungsgefäßes mit den Merkmalen der unabhängigen Ansprüche gelöst. Vorteilhafte Ausführungsformen und Anwendungen der Erfindung ergeben sich aus den abhängigen Ansprüchen.

Gemäß einem ersten Gesichtspunkt der Erfindung wird die oben genannte Aufgabe durch ein Kultivierungsgefäß gelöst, das zur Kultivierung biologischer Zellen in hängenden Tropfen eingerichtet ist und eine Gefäßwand mit einem Deckabschnitt und einem Bodenabschnitt aufweist. Der Deckabschnitt ist für die Bereitstellung einer Kultivierungsfläche zur Aufnahme der hängenden Tropfen eingerichtet. Die Kultivierungsfläche hat eine Oberfläche, die bei Anwendung des Kultivierungsgefäßes zur Kultivierung biologischer Zellen vertikal nach unten, d. h. in Gravitationsrichtung, weist. Der Bodenabschnitt ist zur Aufnahme einer Flüssigkeit eingerichtet. Der Bodenabschnitt bildet ein Reservoir für die Flüssigkeit, die z. B. ein wässriges Medium mit biologischen Zellen, insbesondere eine Zellsuspension in einem Kultivierungsmedium, umfasst. Die Deck- und Bodenabschnitte haben eine flächenhafte, vorzugsweise ebene oder zumindest in Teilen gekrümmte Ausdehnung, und sie erstrecken sich beim Gebrauch während der Kultivierung vorzugsweise in einer horizontalen Richtung und/oder mit einem im Wesentlichen konstanten Abstand.

Die Gefäßwand ist so gebildet, dass ein Innenraum des Kultivierungsgefäßes von dem Deckabschnitt und dem Bodenabschnitt allseits eingeschlossen wird. Der Innenraum ist durch die Gefäßwand von der Umgebung abgegrenzt. Vorteilhafterweise ermöglicht dies eine lokale Einstellung von Kultivierungsbedingungen, wie z. B. die Bereitstellung eines gasförmigen Kultivierungsmediums, einer bestimmten Luftfeuchtigkeit und/oder einer bestimmten Temperatur im Kultivierungsgefäß.

Gemäß der Erfindung ist die Kultivierungsfläche mit Halteelementen ausgestattet, die für eine Positionierung der hängenden Tropfen eingerichtet sind. Die Halteelemente sind so gebildet, dass bei deren Benetzung mit der Kultivierungsflüssigkeit an den Halteelementen eine Sammlung von Flüssigkeit und eine Bildung der hängenden Tropfen unterstützt werden. Der Innenraum ist so gebildet, dass die Kultivierungsfläche die Tropfen frei hängend aufnehmen kann. Vorzugsweise weisen der Deckabschnitt und der Bodenabschnitt einen gegenseitigen Arbeitsabstand auf, in dem die Tropfen an der Kultivierungsfläche im Innenraum des Kultivierungsgefäßes frei hängen können, ohne Flüssigkeit am Bodenabschnitt zu berühren.

Des Weiteren ist gemäß der Erfindung die Gefäßwand des Kultivierungsgefäßes beweglich gebildet, so dass die Halteelemente mit der Flüssigkeit vom Bodenabschnitt benetzt werden können. Die Beweglichkeit der Gefäßwand bedeutet, dass Teile der Gefäßwand relativ zueinander bewegt werden können. Die Beweglichkeit der Gefäßwand bedeutet ferner, dass die Flüssigkeit vom Bodenabschnitt temporär mit dem Deckabschnitt in Kontakt gelangt. Da das Kultivierungsgefäß gemäß der Erfindung einen geschlossenen Innenraum aufweist, kann bei der Bewegung des Kultivierungsgefäßes eine Bewegung der Flüssigkeit erfolgen, ohne dass diese in die Umgebung austritt. Ruckartige Bewegungen des Kultivierungsgefäßes sind abweichend von der oben beschriebenen manuellen Technik vermeidbar. Eventuell unerwünschte Scherkräfte in der Flüssigkeit können minimiert oder ausgeschlossen werden.

Gemäß einem zweiten Gesichtspunkt der Erfindung wird die oben genannte Aufgabe durch ein Verfahren zur Kultivierung biologischer Zellen in hängenden Tropfen gelöst, bei dem das Kultivierungsgefäß gemäß dem ersten Gesichtspunkt der Erfindung verwendet wird. Eine Flüssigkeit, insbesondere eine Suspension, welche die biologischen Zellen in einem flüssigen Medium enthält, wird am Bodenabschnitt des Kultivierungsgefäßes bereitgestellt. Die Gefäßwand des Kultivierungsgefäßes wird derart bewegt, dass die Kultivierungsfläche durch die Suspension mit den biologischen Zellen benetzt wird. Anschließend erfolgt eine Rückstellung der Gefäßwand, wobei die Kultivierungsfläche von der Suspension am Bodenabschnitt getrennt wird und Tropfen der Suspension mit den Zellen an den Halteelementen gesammelt werden. Anschließend folgt die Kultivierung (Vermehrung und/oder Differenzierung) der biologischen Zellen in den hängenden Tropfen, wobei z. B. Zellaggregate und/oder differenzierte Zellen gebildet werden.

Die Erfindung bietet gegenüber den herkömmlichen Techniken eine Reihe von Vorteilen. Die Beweglichkeit der geschlossenen Gefäßwand ermöglicht eine Beladung der Kultivierungsfläche ohne ein ruckartiges Verschwenken des Kultivierungsgefäßes.

Die Benetzung der Kultivierungsfläche kann mit einer minimal bewegten oder ruhenden Zellsuspension erfolgen. Bei der Bildung der hängenden Tropfen können Scherkräfte ausgeschlossen oder auf ein unschädliches Maß minimiert werden. Das erfindungsgemäße Kultivierungsgefäß erlaubt eine schonende Überführung der Zellen aus dem suspendierten Zustand in den hängenden Tropfen. Ein weiterer Vorteil des erfindungsgemäßen Kultivierungsgefäßes ergibt sich daraus, dass von der Gefäßwand der geschlossene Innenraum gebildet wird. Einflüsse aus der Umgebung werden minimiert. Eine Einstellung der Kultivierungsbedingungen im Innenraum wird vereinfacht. Unerwünschte Kontaminationen werden ausgeschlossen. Schließlich ermöglicht das erfindungsgemäße Kultivierungsgefäß eine Automatisierung der Kultivierung biologischer Zellen in hängenden Tropfen. Der gesamte Prozess der Kultivierung, beginnend mit der Bereitstellung der Zellsuspension und der Bildung der hängenden Tropfen und ferner mit der Vermehrung und/oder Differenzierung der Zellen in den hängenden Tropfen, kann automatisiert werden. Eine manuelle Betätigung des Kultivierungsgefäßes zur Beladung der Kultivierungsfläche ist möglich, jedoch nicht vom Geschick des Bedieners abhängig und daher einfach automatisierbar.

Die Kultivierungsfläche des erfindungsgemäßen Kultivierungsgefäßes ist mit Halteelementen ausgestattet, welche die Positionen der hängenden Tropfen bestimmen. Gemäß einer bevorzugten Ausführungsform der Erfindung umfassen die Halteelemente hydrophile Oberflächenbereiche (hydrophile Inseln, hydrophile Spots) der Kultivierungsfläche, die voneinander durch hydrophobe Oberflächenbereiche getrennt sind. Die hydrophilen Oberflächenbereiche ermöglichen vorteilhafterweise eine zuverlässige Sammlung der Tropfen wässriger Medien. Die Größe der Tropfen kann durch die Größe der hydrophilen Oberflächenbereiche beeinflusst werden.

Vorteilhafterweise bestehen verschiedene Möglichkeiten der Gestaltung der Halteelemente. Wenn die Kultivierungsfläche beispielsweise durch eine Oberfläche einer Folie gebildet wird, können die hydrophilen und/oder die hydrophoben Oberflächenbereiche durch eine Funktionalisierung der Folienoberfläche bereitgestellt werden. Alternativ oder zusätzlich können gemäß einer weiteren Variante die Halteelemente hydrophile Stufenelemente, insbesondere lokale Vertiefungen oder Vorsprünge, der Kultivierungsfläche umfassen, an denen die Wirkung von Kapillarkräften größer als in unstrukturierten Oberflächenbereichen der Kultivierungsfläche ist. Wenn die Kultivierungsfläche beispielsweise durch die Oberfläche einer Kunststoffplatte oder -folie gebildet wird, können die hydrophilen Stufenelemente durch ring-, quader- oder zylinderförmige Vorsprünge mit typischen Dimensionen (Querschnitt, Höhe) im Sub-mm-Bereich gebildet werden. Besonders bevorzugt umfassen die hydrophilen Stufenelemente umlaufende Vorsprünge, z. B. in Gestalt von Ringen. Umlaufende, z. B. ringförmige Vorsprünge können eine Doppelfunktion erfüllen: während der Kultivierung von Zellen oder Zellaggregaten in den hängenden Tropfen bilden die Vorsprünge eine Abgrenzung in Bezug auf benachbarte hängende Tropfen, und bei einer optional vorgesehenen weiteren Kultivierung im adhärenten Zustand bilden sie eine Aufnahme für ein Kultivierungsmedium.

Vorzugsweise sind die Halteelemente, insbesondere die hydrophilen Oberflächenbereiche, mit einem regelmäßigen Muster, z. B. einer Matrixanordnung mit geraden Reihen und Spalten der Halteelemente, angeordnet. Vorteilhafterweise werden dadurch die Identifizierung von Proben in einzelnen hängenden Tropfen und die Automatisierung der erfindungsgemäßen Kultivierung in hängenden Tropfen vereinfacht.

Vorteilhafterweise kann die Bewegung der Gefäßwand zur Benetzung der Kultivierungsfläche erfolgen, ohne dass das gesamte Kultivierungsgefäß geschwenkt wird. Gemäß der Erfindung weist das Kultivierungsgefäß eine deformierbare Gefäßwand auf. Der Deckabschnitt und der Bodenabschnitt sind relativ zueinander beweglich. Bei einer Deformation der Gefäßwand (Kompression des Kultivierungsgefäßes) ist der Abstand des Deckabschnittes und des Bodenabschnittes verringerbar, so dass die Kultivierungsfläche mit den Haltelementen an den Bodenabschnitt angenähert werden kann. Vorteilhafterweise ermöglicht die Deformierbarkeit der Gefäßwand, dass die Kultivierungsfläche und der Bodenabschnitt so zueinander bewegt werden können, dass die Halteelemente mit der Flüssigkeit, die am Bodenabschnitt aufgenommen ist, in Kontakt geraten. Die Gefäßwand ist so deformierbar, dass die Kultivierungsfläche mit der Flüssigkeit am Bodenabschnitt benetzt, z. B. in die Flüssigkeit eingetaucht, werden kann.

Bei dieser Ausführungsform der Erfindung umfasst die Bildung der hängenden Tropfen zunächst eine Kompression des Kultivierungsgefäßes, um die Halteelemente mit der Flüssigkeit zu benetzen. Anschließend erfolgt eine Rückstellung der Gefäßwand, wobei sich der Abstand des Deckabschnitts und des Bodenabschnitts wieder vergrößert, bis zwischen diesen ein gewünschter Arbeitsabstand zur Aufnahme der hängenden Tropfen erreicht wird. Während der Rückstellung der Gefäßwand wird die Kultivierungsfläche von der Suspension am Bodenabschnitt getrennt, wobei sich Suspension mit den Zellen an den Halteelementen sammelt, so dass an den Halteelementen die hängenden Tropfen gebildet werden.

Die Gefäßwand des erfindungsgemäßen Kultivierungsgefäßes kann aus mehreren Materialien zur Bereitstellung der Deck- und Bodenabschnitte oder alternativ einstückig aus einem einzigen Material hergestellt sein. Es bestehen verschiedene Möglichkeiten der Materialauswahl, um die gewünschte Deformierbarkeit der Gefäßwand bereitzustellen. Gemäß einer bevorzugten Ausführungsform der Erfindung kann die Gefäßwand aus einem biegsamen Folienmaterial hergestellt sein. In diesem Fall bildet das Kultivierungsgefäß einen flexiblen Beutel in Gestalt eines abgeplatteten Kissens, das sich bei Gebrauch des Kultivierungsgefäßes im Wesentlichen in horizontaler Richtung erstreckt. Die Deck- und Bodenflächen des Kultivierungsgefä-βes werden durch die Hauptflächen des Beutels bereitgestellt. Der Abstand zwischen der Kultivierungsfläche und dem Bodenabschnitt, insbesondere eine lichte Weite des Innenraums im Kultivierungsgefäß aus dem biegsamen Folienmaterial kann unter der Wirkung eines Innendruckes im Kultivierungsgefäß und/oder unter Verwendung eines faltbaren Innenträgers eingestellt werden. Der Innendruck kann durch eine Pumpeinrichtung oder durch eine Verminderung des Volumens des Innenraums, z. B. an einem Wickelabschnitt oder einem Quetschabschnitt des Kultivierungsgefäßes eingestellt werden. Der faltbare Innenträger kann ein zusammenlegbares Gestell im Innenraum des Kultivierungsgefäßes umfassen.

Gemäß einer alternativen Ausführungsform der Erfindung ist die Gefäßwand zumindest teilweise aus einem elastisch deformierbaren Material hergestellt. In diesem Fall kann der Arbeitsabstand zwischen den Deck- und Bodenabschnitten unter der Wirkung einer inneren elastischen Rückstellkraft des elastisch deformierbaren Materials einstellbar sein. Vorzugsweise ist das elastisch deformierbare Material im Deckabschnitt und/oder im Bodenabschnitt in Randbereichen vorgesehen, in denen die Verbindung mit dem Bodenabschnitt bzw. dem Deckabschnitt gebildet wird. Das elastische deformierbare Material bildet vorzugsweise einen Seitenabschnitt, über den die Deck- und Bodenabschnitte seitlich, d. h. lateral in Bezug auf die Kultivierungsfläche und die Zellsuspension am Bodenabschnitt, miteinander verbunden sind.

Gemäß einer weiteren Variante der Erfindung kann die Gefäßwand aus einem biegsamen Folienmaterial und einem elastisch deformierbaren Material zusammengesetzt sein. Beispielsweise kann das biegsame Folienmaterial entlang der lateralen Ausdehnung der Deck- und Bodenabschnitte vorgesehen sein, während das elastisch deformierbare Material in den Randbereichen zwischen den Deck- und Bodenabschnitten, insbesondere den Seitenabschnitt bildend, vorgesehen ist. Des Weiteren kann die Gefäßwand vollständig oder teilweise aus einem elastischen, biegsamen Folienmaterial hergestellt sein.

Gemäß einer weiteren Variante der Erfindung kann die Gefäßwand teilweise aus einem starren, plattenförmigen Wandmaterial hergestellt sein. Das plattenförmige Wandmaterial kann z. B. die Deck- und/oder Bodenabschnitte entlang von deren lateraler Ausdehnung bilden, während Randbereiche der Deck- und Bodenabschnitte über den Seitenabschnitt aus dem elastisch deformierbaren Material und/oder dem biegsamen Folienmaterial gebildet sind.

Vorzugsweise ist der Bodenabschnitt so gebildet, dass die Flüssigkeit im Kultivierungsgefäß den Bodenabschnitt vollständig bedecken kann. Dadurch kann die gesamte Kultivierungsfläche im komprimierten Zustand des Kultivierungsgefäßes benetzt werden. Die Wirksamkeit der Bildung hängender Tropfen wird verbessert. Gemäß einer abgewandelten Ausführungsform der Erfindung kann der Bodenabschnitt eine zur Kultivierungsfläche im Innenraum des Kultivierungsgefäßes gegenüberliegende Bodenfläche aufweisen, die für eine lokal selektive Bereitstellung der Flüssigkeit an hydrophilen Oberflächenbereichen eingerichtet ist, die durch hydrophobe Oberflächenbereiche voneinander getrennt sind. Die hydrophilen Oberflächenbereiche der Bodenfläche sind mit derselben geometrischen Anordnung wie die Halteelemente der Kultivierungsfläche positioniert. Die Positionen der hydrophilen Oberflächenbereiche der Bodenfläche passen zu den Positionen der Halteelemente der Kultivierungsfläche. Vorteilhafterweise kann damit bei der Bildung der hängenden Tropfen der Flüssigkeitsverbrauch deutlich vermindert werden. Im Bodenabschnitt wird die Flüssigkeit, insbesondere die Zellsuspension, nur in den hydrophilen Oberflächenbereichen und damit an den Positionen bereitgestellt, an denen bei Annäherung der Kultivierungsfläche hängende Tropfen gebildet werden können.

Vorzugsweise wird die Kultivierungsfläche des Kultivierungsgefäßes unmittelbar durch eine Innenfläche des Deckabschnittes gebildet. Vorteilhafterweise können in diesem Fall die hängenden Tropfen direkt an der Innenseite der Gefäßwand gebildet werden. Eine Beobachtung und ggf. Manipulation der Tropfen und der Aufbau des Kultivierungsgefäßes werden vereinfacht. Alternativ kann die Kultivierungsfläche an einer anderen inneren Oberfläche im Innenraum des Kultivierungsgefäßes vorgesehen sein.

Gemäß einer weiteren vorteilhaften Ausführungsform der Erfindung kann das Kultivierungsgefäß mindestens eine Zwischenwand aufweisen, durch die der Innenraum in mindestens zwei Horizontalkammern unterteilt wird. Mit der Bereitstellung von mindestens zwei Horizontalkammern werden weitere Anwendungen des Kultivierungsgefäßes und komplexe Kultivierungsverfahren ermöglicht. Die Zwischenwand kann z. B. ein Material, das eine molekulare Diffusion, insbesondere von biologisch wirksamen Molekülen, wie z. B. Wachstums- oder Differenzierungsfaktoren erlaubt, ein poröses Material und/oder ein Material mit Solbruchstellen aufweisen. Vorteilhafterweise ermöglicht die Zwischenwand, dass eine zunächst ausschließlich in einer der Horizontalkammern vorhandene Substanz zu Beginn und/oder während der Kultivierung durch Diffusion und/oder eine Freisetzung durch die Poren und/oder die Sollbruchstellen in die andere Horizontalkammer überführt wird. Damit werden zusätzliche Freiheitsgrade für die Kultivierung der biologischen Zellen geschaffen. Durch die Zwischenwand kann z. B. ein molekularer Differenzierungsfaktor diffundieren, mit dem die Differenzierung biologischer Zellen in hängenden Tropfen beeinflusst wird.

Die Zwischenwand kann sich über den gesamten Innenraum erstrecken, um zwei getrennte Horizontalkammern zu bilden. In diesem Fall ist die Zwischenwand z. B. als Teil des Bodenabschnitts vorgesehen, um die Flüssigkeit im Bodenabschnitt erst nach Öffnung von Sollbruchstellen freizugeben oder die Flüssigkeit im Bodenabschnitt zeitlich verzögert mit einer biologisch wirksamen Substanz zu versetzen. Alternativ kann die Zwischenwand Teil des Deckabschnitts sein, um in diesem auf der zum Bodenabschnitt weisenden Seite die Kultivierungsfläche zu bilden. In diesem Fall können von der Horizontalkammer, welche durch die Zwischenwand vom übrigen Innenraum des Kultivierungsgefäßes abgetrennt ist, biologisch wirksame Substanzen in die hängenden Tropfen diffundieren.

Gemäß einer weiteren vorteilhaften Ausführungsform der Erfindung ist das Kultivierungsgefäß mit einer Trägereinrichtung ausgestattet, die das Kultivierungsgefäß an seiner Unterseite abstützt. Die Trägereinrichtung befindet sich an der bei Gebrauch des Kultivierungsgefäßes in Gravitationsrichtung weisenden Seite, insbesondere am Bodenabschnitt des Kultivierungsgefäßes. Vorteilhafterweise erlaubt die Trägereinrichtung eine stabile Positionierung des Kultivierungsgefäßes auf einer Plattform, wie z. B. einem Labortisch, oder in einer automatisierten Kultivierungsanlage. Vorzugsweise umfasst die Trägereinrichtung Stützfüße, die am Bodenabschnitt des Kultivierungsgefäßes verteilt angeordnet sind.

Wenn die Trägereinrichtung gemäß einer weiteren Variante der Erfindung Massenelemente aufweist, mit denen ein Massenschwerpunkt des Kultivierungsgefäßes, insbesondere im Gebrauch, im Bodenabschnitt oder an diesen angrenzend, gebildet wird, kann die Trägereinrichtung vorteilhafterweise zusätzlich eine stabilisierende Funktion erfüllen. Insbesondere bei Verwendung einer Gefäßwand, die aus einer biegsamen Folie hergestellt ist, wird das Kultivierungsgefäß mit den Massenelementen der Trägereinrichtung an der Unterseite stabilisiert. Eine unerwünschte unregelmäßige Deformation z. B. eines beutelförmigen Kultivierungsgefäßes und damit eine unerwünschte Bewegung der Zellsuspension werden vermieden.

Gemäß der Erfindung hat das Kultivierungsgefäß einen geschlossenen Innenraum, der von den Deck- und Bodenabschnitten, gegebenenfalls in Kombination mit dem Seitenabschnitt, allseits begrenzt wird. Um eine Beeinflussung der Kultivierung biologischer Zellen im geschlossenen Innenraum zu vereinfachen, ist das Kultivierungsgefäß gemäß einer weiteren Ausführungsform der Erfindung vorzugsweise mit einer Medieneinrichtung mit mindestens einer verschließbaren Medien-Schnittstelle, besonders bevorzugt mindestens zwei Medien-Schnittstellen, ausgestattet, die zur Zu- und Abfuhr von flüssigen und/oder gasförmigen Medien in und aus dem Innenraum eingerichtet ist. Die mindestens eine Medien-Schnittstelle umfasst beispielsweise einen Schlauchanschluss, eine Öffnung mit einem Deckel, oder einen Wandabschnitt, der von Leitungen für die flüssigen und/oder gasförmigen Medien durchbohrt werden kann.

Vorteilhafterweise ist die Gefäßwand des Kultivierungsgefäßes gemäß einer weiteren Abwandlung der Erfindung mit mindestens einem Fensterabschnitt ausgestattet, der einen optischen und/oder mechanischen Zugriff auf mindestens einen der hängenden Tropfen erlaubt. Gemäß einer Variante kann der mindestens eine Fensterabschnitt für eine optische Beobachtung des mindestens einen hängenden Tropfens ausgelegt sein. In diesem Fall umfasst der mindestens eine Fensterabschnitt vorzugsweise einen ebenen, plattenförmigen Bereich, der aus einem optisch transparenten Material hergestellt ist, im Deckabschnitt. Alternativ oder zusätzlich kann der mindestens eine Fensterabschnitt für eine invasive Durchbrechung durch ein Werkzeug ausgelegt sein. Hierzu kann der Fensterabschnitt z. B. eine Sollbruchstelle im Deckabschnitt aufweisen, die an die Position eines Halteelements an der Kultivierungsfläche angrenzend angeordnet ist.

Wenn eine deformierbare Gefäßwand verwendet wird, sind Ausführungsformen des Kultivierungsgefäßes möglich, bei denen die äußere Form des Kultivierungsgefäßes durch mechanische Einflüsse veränderlich ist. Zur Vermeidung unerwünschter Deformationen ist gemäß einer weiteren vorteilhaften Ausführungsform der Erfindung eine äußere Aufnahmeeinrichtung für das Kultivierungsgefäß vorgesehen, die zur Aufnahme der Gefäßwand eingerichtet ist. Die äußere Aufnahmeeinrichtung, z. B. in Gestalt eines Kastens, gegebenenfalls mit Durchbrechungen zur Durchführung von Medienleitungen, bildet einen Schutz und eine Anlagefläche für die Gefäßwand. Vorzugsweise weist die äußere Aufnahmeeinrichtung eine transparente Platte auf, an welcher der Deckabschnitt anliegt, wenn im Gebrauch des Kultivierungsgefäßes der Arbeitsabstand zwischen den Deck- und Bodenabschnitten eingestellt ist und die Kultivierung der Zellen in den hängenden Tropfen erfolgt. Die transparente Platte bildet eine ebene Begrenzung des Deckabschnitts, z. B. in Gestalt einer nachgiebigen Folie, wodurch eine Beobachtung der hängenden Tropfen mit einer optischen Beobachtungseinrichtung vereinfacht wird.

Alternativ oder zusätzlich kann das Kultivierungsgefäß gemäß einer weiteren Modifikation der Erfindung mit einer äußeren Klemmeinrichtung ausgestattet sein. Die Klemmeinrichtung ist in einem Randbereich der Gefäßwand angeordnet, um das Volumen des Innenraums des Kultivierungsgefäßes einzustellen. Bei Betätigung der äußeren Klemmeinrichtung kann das Volumen des Innenraums, beispielsweise durch ein Aufwickeln oder Quetschen eines Teils der Gefäßwand vermindert werden, wobei sich der Druck im Innenraum erhöht.

Gemäß einer weiteren vorteilhaften Ausführungsform der Erfindung sind die Deck- und Bodenabschnitte mit linienförmigen Koppelelementen ausgestattet. Bei einer Deformation des Kultivierungsgefäßes derart, dass sich die Deck- und Bodenabschnitte berühren, kann entlang der linienförmigen Koppelelemente eine Verbindung der Deck- und Bodenabschnitte bereitgestellt werden. Vorteilhafterweise ermöglicht dies die Unterteilung des Innenraums des Kultivierungsgefäßes in Vertikalkammern, in denen beispielsweise verschiedene Kultivierungsbedingungen einstellbar sind.

Das erfindungsgemäße Kultivierungsgefäß hat besondere Vorteile in Bezug auf seine einfache Handhabung und die Bereitstellung eines geschlossenen Systems. Die Trennung des Innenraums von der Umgebung ermöglicht sterile Kultivierungsbedingungen, eine genaue und reproduzierbare Kontrolle der Kultivierungsbedingungen und die Erfüllung von Anforderungen von Kultivierungsprotokollen (wie z.B. Good Manufacturing Practice - GMP). Der Medienwechsel, die Ernte der kultivierten Zellen und die Zugabe von Differenzierungsfaktoren können mit hoher Geschwindigkeit erfolgen, und eine Vielzahl hängender Tropfen kann gleichzeitig gebildet werden. Die erfindungsgemäße Technik ist daher für Anwendungen mit hohem Durchsatz ("high throughput-Verfahren") geeignet.

Weitere Einzelheiten und Vorteile der Erfindung werden im Folgenden unter Bezug auf die beigefügten Zeichnungen beschrieben. Es zeigen:
- Figuren 1A und 1B:: schematische Illustrationen einer ersten Ausführungsform eines erfindungsgemäßen Kultivierungsgefäßes;
- Figuren 2A bis 2C:: eine schematische Illustration der Bildung hängender Tropfen gemäß einer bevorzugten Ausführungsform des erfindungsgemäßen Kultivierungsverfahrens;
- Figuren 3A bis 3E:: weitere Illustrationen von Varianten des erfindungsgemäßen Kultivierungsverfahrens;
- Figuren 4A bis 4D:: eine schematische Illustration einer weiteren Ausführungsform des erfindungsgemäßen Kultivierungsgefäßes, das mit einer Trägereinrichtung ausgestattet ist;
- Figuren 5A bis 5C:: eine schematische Illustration weiterer Varianten des erfindungsgemäßen Verfahrens;
- Figuren 6A bis 6F:: schematische Illustrationen weiterer Ausführungsformen des erfindungsgemäßen Kultivierungsgefäßes;
- Figuren 7A bis 7C:: eine schematische Illustration der optischen Beobachtung hängender Tropfen im erfindungsgemäßen Kultivierungsgefäß;
- Figuren 8A bis 8C:: eine schematische Illustration des Zugriffs auf hängende Tropfen mit einem äußeren Werkzeug;
- Figuren 9A bis 9C:: eine schematische Illustration einer weiteren Ausführungsform eines erfindungsgemäßen Kultivierungsgefäßes in Gestalt eines Schlauches;
- Figuren 10A und 10B:: schematische Illustrationen weiterer Ausführungsformen des erfindungsgemäßen Kultivierungsgefäßes mit Horizontalkammern;
- Figuren 11A bis 11C:: eine schematische Illustration einer weiteren Variante des erfindungsgemäßen Verfahrens;
- Figur 12:: eine schematische Illustration der Wirkung einer äußeren Klemmeinrichtung;
- Figuren 13A bis 13E:: eine schematische Illustration einer weiteren Ausführungsform des erfindungsgemäßen Verfahrens;
- Figuren 14A und 14B:: eine schematische Illustration weiterer Varianten des erfindungsgemäßen Verfahrens; und
- Figuren 15A und 15B:: schematische Illustrationen weiterer Ausführungsformen des erfindungsgemäßen Kultivierungsgefäßes, das mit Koppelelementen zur Bildung von Vertikalkammern ausgestattet ist; und
- Figuren 16 bis 20:: schematische Illustrationen weiterer Ausführungsformen des erfindungsgemäßen Kultivierungsgefäßes, das mit Koppelelementen zur Bildung von Vertikalkammern ausgestattet ist.

Bevorzugte Ausführungsformen der Erfindung werden im Folgenden unter Bezug auf die Gestaltung des Kultivierungsgefäßes und die Ausführung des Verfahrens zur Kultivierung biologischer Zellen beschrieben. Einzelheiten des Kultivierungsverfahrens werden nicht genannt, soweit diese von herkömmlichen Verfahren zur Kultivierung biologischer Zellen für Vermehrungs- und/oder Differenzierungszwecke, insbesondere in hängenden Tropfen, bekannt sind. Kultivierungsbedingungen, insbesondere die Wahl von Kultivierungsmedien, die Gestaltung von Kultivierungsprotokollen (Zu- und Abführung bestimmter Medien nach einem bestimmten Zeitplan) und physikalische Bedingungen, wie z. B. die Temperatur, der Druck, die Luftfeuchtigkeit und die Beleuchtung, können bereitgestellt werden, wie es von herkömmlichen Kultivierungsverfahren bekannt ist. Die Anwendung der Erfindung ist nicht auf die Kultivierung bestimmter Typen von Zellen beschränkt, sondern mit verschiedenartigen Zellen, insbesondere differenzierten Zellen, Stammzellen oder Zellgruppen, anwendbar. Allerdings ist die Kultivierung von humanen embryonalen Stammzellen vom Schutz ausgenommen.

Ausführungsformen der Erfindung werden unter beispielhaftem Bezug auf Kultivierungsgefäße beschrieben, die für eine manuelle Handhabung, z. B. in einem Labor, ausgelegt sind. Die Erfindung ist nicht auf die beispielhaft beschriebenen Formen und Größen beschränkt, sondern entsprechend auch mit Kultivierungsgefäßen anwendbar, die an andere Anwendungen, wie z. B. in automatisierten Kultivierungsanlagen, angepasst sind.

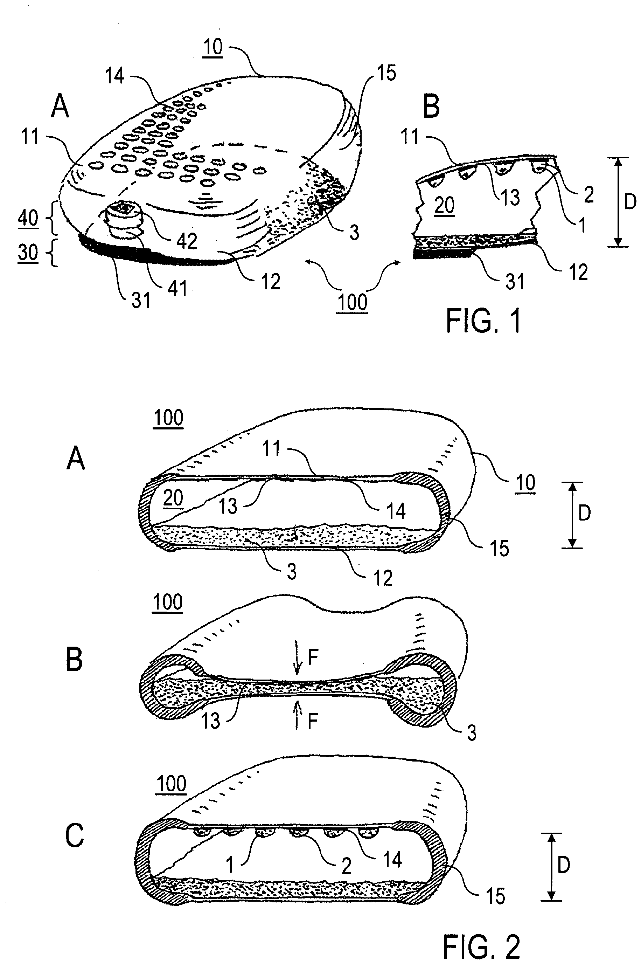

In Figur 1 ist eine erste Ausführungsform des erfindungsgemä-ßen Kultivierungsgefäßes 100 in Perspektivansicht (Figur 1A) und als Ausschnitt in Schnittansicht (Figur 1B) gezeigt. Das Kultivierungsgefäß 100 umfasst eine Gefäßwand 10, die aus einem flexiblen Kunststoffmaterial, insbesondere einer biokompatiblen Polymerfolie, wie z. B. Polyethylen oder Ethylenvinylacetat, hergestellt ist. Die Gefäßwand 10 bildet einen flexiblen Beutel mit einer flächigen, abgeplatteten Gestalt, die sich bei Gebrauch des Kultivierungsgefäßes 100 in horizontaler Richtung erstreckt. An einer ersten Seite (Oberseite) der Gefäßwand 10 bildet die Polymerfolie einen Deckabschnitt 11, und an einer zweiten Seite (Unterseite) einen Bodenabschnitt 12. Der Bodenabschnitt 12 hat die Gestalt einer flachen Wanne, die zur Aufnahme einer Flüssigkeit 3 (Zellsuspension) vorgesehen ist. Der gekrümmte Wandabschnitt, an dem die Deck- und Bodenabschnitte 11, 12 miteinander verbunden sind, wird als Seitenabschnitt 15 bezeichnet. Von den Deck-, Boden- und Seitenabschnitten 11, 12, 15 wird ein Innenraum 20 allseits eingeschlossen, in dem die Kultivierung biologischer Zellen in hängenden Tropfen erfolgt. Die Deck- und Bodenabschnitte 11, 12 sind im Wesentlichen flächig ausgedehnte Wandabschnitte mit einem gegenseitigen Abstand D, die an ihren Rändern, hin zum Seitenabschnitt 15, gekrümmt sind.

Die Innenfläche des Deckabschnitts 11 bildet die Kultivierungsfläche 13, die für die Kultivierung der biologischen Zellen in hängenden Tropfen vorgesehen ist. Auf der Kultivierungsfläche 13 sind Halteelemente 14 angeordnet. Die Halteelemente 14 sind für die Positionierung der Tropfen 2 (siehe Figur 1B) eingerichtet und über die gesamte Kultivierungsfläche 13 verteilt angeordnet (in Figur 1A teilweise gezeigt). Die Anordnung der Halteelemente 14 umfasst z. B. eine regelmäßige Matrixanordnung mit geraden Reihen und Spalten.

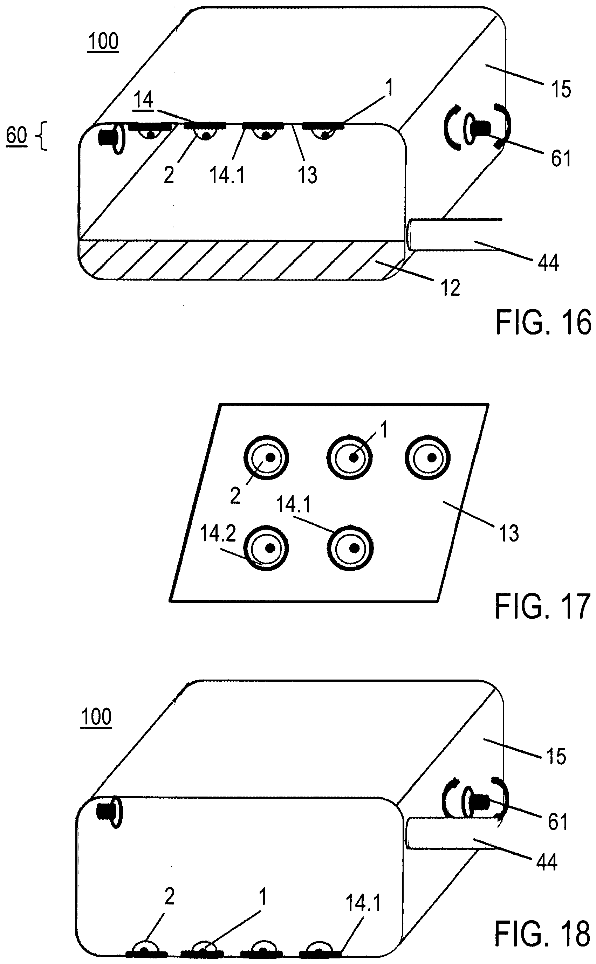

Bei der dargestellten Ausführungsform des Kultivierungsgefä-βes 100 umfassen die Halteelemente 14 hydrophile Oberflächenbereiche der Kultivierungsfläche 13, die durch die im Übrigen hydrophoben Oberfläche der Kultivierungsfläche 13 voneinander getrennt sind. Die lokalen hydrophilen Oberflächenbereiche sind entlang der Kultivierungsfläche 13 von der hydrophoben Oberfläche allseits umgeben. Alternativ oder zusätzlich können die Halteelemente 14 stufenförmige Mikrostrukturen umfassen, die eine flüssigkeitsanziehende Wirkung haben (siehe Figuren 16 bis 20).

Das Kultivierungsgefäß 100 ist mit einer Trägereinrichtung 30 ausgestattet, die auf der Außenseite der Gefäßwand 10, insbesondere auf der Außenseite des Bodenabschnitts 12 angeordnet ist. Die Trägereinrichtung 30 umfasst ein ringförmiges Massenelement 31, das z. B. aus einem Kunststoffmaterial, optional mit einer Metalleinlage, hergestellt ist. Mit dem Massenelement 31 ruht das Kultivierungsgefäß 100 auf einer Unterlage, z. B. einem Labortisch, oder in einer Halterung (nicht gezeigt). Das Massenelement 31 ist so gebildet, dass der Massenschwerpunkt des Kultivierungsgefäßes 100 im Bodenabschnitt 12 oder an diesen angrenzend liegt. Dies ermöglicht, dass das Kultivierungsgefäß 100, obwohl es aus einem deformierbaren, flexiblen Beutel hergestellt ist, nach einer Bewegung einfach in eine Lage gebracht werden kann, in der sich der Deckabschnitt 11 auf der nach oben (entgegengesetzt zur Gravitationsrichtung) weisenden Seite des Kultivierungsgefäßes 100 befindet.

Das Kultivierungsgefäß 100 ist des Weiteren mit einer Medien-Einrichtung 40 ausgestattet, die bei der dargestellten Ausführungsform eine Einfüllöffnung 41 mit einem Deckel 42 umfasst. Der Deckel 42 kann, z. B. für Identifizierungszwecke, eine Markierung, wie z. B. einen optischen Code (Barcode, QR-Code) tragen. Die Einfüllöffnung 41 umfasst ein Ansatzrohrstück, z. B. aus Kunststoffmaterial, das mit dem Seitenabschnitt 15 fest verbunden ist und den Deckel 41 trägt.

Das Kultivierungsgefäß 100 weist zwei Betriebszustände auf. In einem ersten Betriebszustand (entfalteter Zustand) ist die Gefäßwand 10 aufgespannt, so dass zwischen dem Deckabschnitt 11 und dem Bodenabschnitt 12 ein Arbeitsabstand D gebildet ist, der mindestens doppelt so groß wie der Durchmesser der hängenden Tropfen 2, insbesondere größer als 5 mm, z. B. 100 mm ist. Im entfalteten Zustand des Kultivierungsgefäßes 100 können die Tropfen 2 mit den biologischen Zellen 1 frei an der nach unten weisenden Kultivierungsfläche 13 des Deckabschnitts 11 hängen, ohne andere Teile im Innenraum 20, insbesondere die Flüssigkeit 3 im Bodenabschnitt 12, zu berühren. In einem zweiten Betriebszustand (komprimierter Zustand) ist die Gefäßwand 10 deformiert, so dass die Kultivierungsfläche 13 die Flüssigkeit 3 im Bodenabschnitt 12 berührt. Im komprimierten Zustand wird die Kultivierungsfläche 13 mit der Flüssigkeit 3 benetzt. Zwischen den beiden Betriebszuständen kann durch die Ausübung einer auf die Gefäßwand 10, insbesondere im Innenraum 20 oder in dem Seitenabschnitt 15 wirkende Kraft, gewechselt werden. Im komprimierten Zustand erfolgt die Beladung der Kultivierungsfläche 13 mit der Flüssigkeit 3, während im entfalteten Zustand die Kultivierung der Zellen 1 in den hängenden Tropfen erfolgt.

Die erfindungsgemäße Kultivierung der biologischen Zellen 1 in den hängenden Tropfen 2, insbesondere mit dem Kultivierungsgefäß 100 gemäß Figur 1, umfasst die folgenden Schritte. Das Kultivierungsgefäß 100 ruht auf einer Trägerplattform (nicht dargestellt). Zuerst wird die Flüssigkeit 3, umfassend eine Zellsuspension z. B. humane pluripotente-Stammzellen in Kulturmedium, am Bodenabschnitt 12 des Kultivierungsgefäßes 100 bereitgestellt. Die Zellsuspension wird durch die Einfüllöffnung 41 in den Bodenabschnitt 12 geleitet, so dass dieser mit einer Flüssigkeitsschicht der Dicke von z. B. 10 mm bedeckt ist. Anschließend wird der Innenraum 20 durch die Einfüllöffnung 41 mit einem gasförmigen Medium, z. B. ein Luft-CO₂-Gemisch mit 5% CO₂ beaufschlagt, so dass der entfaltete Zustand des Kultivierungsgefäßes 100 gebildet wird. Aufgrund einer Reststeifigkeit der Polymerfolie der Gefäßwand 10 bleibt der entfaltete Zustand bis zum Verschluss der Einfüllöffnung 41 mit dem Deckel 42 erhalten.

Nach dem druckdichten Verschluss der Einfüllöffnung 41 mit dem Deckel 42 erfolgt eine Kompression des Kultivierungsgefä-βes 100 derart, dass sich der Abstand zwischen dem Deckabschnitt und dem Bodenabschnitt 12 verringert. Hierzu wird beispielsweise manuell oder mit einem Werkzeug auf die Oberseite des Kultivierungsgefäßes 100 gedrückt. Die Flexibilität des Materials der Gefäßwand 10 und die Komprimierbarkeit des im Innenraum 20 vorhandenen Gases erlaubt ein Eintauchen der Kultivierungsfläche 13 in die Flüssigkeit 3. Nach der Benetzung der Kultivierungsfläche 13 erfolgt eine Rückstellung der Gefäßwand 10, wobei unter der Wirkung des Innendrucks im Innenraum 20 der Arbeitsabstand D zwischen dem Deckabschnitt 11 und dem Bodenabschnitt 12 hergestellt wird. Die an der Kultivierungsfläche 13 anhaftende Flüssigkeit 3 wird an den Halteelementen 14 gesammelt, wo sich die hängenden Tropfen 2 bilden. Anschließend folgt die Kultivierung der Zellen 1 in den hängenden Tropfen 2 entsprechend dem gewünschten Kultivierungsprotokoll. Im geschlossenen Innenraum 20 herrscht über der Flüssigkeit 3 eine mit Wasserdampf gesättigte Atmosphäre, so dass die Zellen 1 in den hängenden Tropfen 2 über Tage oder Woche kultiviert werden können, ohne dass die hängenden Tropfen sich durch Verdunstung verkleinern.

In einem praktischen Beispiel weist das Kultivierungsgefäß 100 z. B. die folgenden Dimensionen auf: Deckabschnitt 11 und Bodenabschnitt 12: 20 cm . 20 cm, Arbeitsabstand D: 3 cm, Flüssigkeitsvolumen im Bodenabschnitt 12: 80 ml, Durchmesser der hängenden Tropfen 2: 3 mm, Anzahl der hängenden Tropfen 2: 150.

Die Bildung des entfalteten Zustands des Kultivierungsgefäßes 100 durch einen erhöhten Innendruck im Innenraum 20 ist nicht zwingend erforderlich. Ersatzweise kann der entfaltete Zustand unter Benutzung einer elastischen Rückstellkraft eingestellt werden, die vom Material der Gefäßwand 10, insbesondere im Seitenabschnitt 15, erzeugt wird. Diese Ausführungsform der Erfindung ist schematisch in Figur 2 gezeigt. Die Figuren 2A und 2C illustrieren den entfalteten Zustand, während Figur 2B den komprimierten Zustand des Kultivierungsgefäßes 100 in perspektivischer, geschnittener Ansicht illustriert.

Das Kultivierungsgefäß 100 gemäß Figur 2 umfasst, wie oben beschrieben, einen Deckabschnitt 11 zur Bereitstellung der Kultivierungsfläche 13 für die Aufnahme der hängenden Tropfen 2, einen Bodenabschnitt 12 zur Aufnahme der Flüssigkeit 3 und einen Seitenabschnitt 15. Die Abschnitte 11, 12 und 15 schließen den Innenraum 20 ein. Die Deck- und Bodenabschnitte 11, 12 sind im entfalteten Zustand ebene Schichten aus einem flexiblen Material, z. B. deformierbare Kunststofffolien. Die ebene Form der Deck- und Bodenabschnitte 11, 12 wird durch deren eigene Formhaltigkeit und dadurch gebildet, dass sie durch den Seitenabschnitt 15 aufgespannt werden. Der Seitenabschnitt 15 ist aus einem elastischen Material, z. B. Silikon hergestellt, das eine innere Vorspannung aufweist und eine elastische Rückstellkraft auf die Gefäßwand 10 ausübt. Das Kultivierungsgefäß 100 ist mit einer Medien-Einrichtung ausgestattet, die eine Einfüllöffnung gemäß Figur 1 oder eine andere Medien-Schnittstelle (siehe unten) umfassen kann (in gemäß Figur 2 nicht dargestellt). Des Weiteren kann das Kultivierungsgefäß 100 gemäß Figur 2 mit einer Trägereinrichtung zur Unterstützung des Kultivierungsgefäßes 100 ausgestattet sein (nicht dargestellt).

Figur 2A zeigt das Kultivierungsgefäß 100 zu Beginn des Kultivierungsverfahrens. Die Flüssigkeit 3, insbesondere eine Zellsuspension, ist im Bodenabschnitt 12 angeordnet. Die Kultivierungsfläche 13 weist durch die aufspannende Wirkung des Seitenabschnitts 15 den Arbeitsabstand D vom Bodenabschnitt 12 auf. Die Flüssigkeit 3 befindet sich aufgrund ihrer Schwerkraft auf der Innenseite des Bodenabschnitts 12.

Figur 2B zeigt den komprimierten Zustand des Kultivierungsgefäßes 100. Unter der Wirkung einer äußeren Kraft F, die auf mindestens einen der Deck- und Bodenabschnitte 11, 12 wirkt, wird die Kultivierungsfläche 13 auf der Innenseite des Deckabschnitts 11 in die Flüssigkeit 3 eingetaucht. Die Kultivierungsfläche 13 wird benetzt, so dass sich die Flüssigkeit 3 an den Halteelementen 14 (hydrophile Oberflächenbereiche der Kultivierungsfläche 13) sammeln kann.

Bei der Bereitstellung des komprimierten Zustandes wird die von außen wirkende Kraft F z. B. manuell oder mit einer äußeren Stelleinrichtung (nicht dargestellt) ausgeübt. Die Verwendung einer äußeren Stelleinrichtung hat den Vorteil, dass diese eine kontrollierte, stufenlose Bewegung beim Komprimieren und bei der Rückstellung ermöglicht. Die stufenlose Bewegung wirkt sich vorteilhaft auf eine Vermeidung des Abfallens von Tropfen während der Rückstellbewegung aus.

Nach Freigabe der Einwirkung der äußeren Kraft F erfolgt die Rückstellung des Kultivierungsgefäßes 100 in den entfalteten Zustand (Figur 2C). Die Rückstellung wird durch die elastische Rückstellkraft des Seitenabschnitts 15 bewirkt. An den Halteelementen 14 haben sich die hängenden Tropfen 2 mit den biologischen Zellen 1 gesammelt. Im entfalteten Zustand erfolgt die weitere Kultivierung der Zellen 1 in den hängenden Tropfen 2 entsprechend dem gewünschten Kultivierungsprotokoll.

Ein besonderer Vorteil der Ausführungsform gemäß Figur 2 besteht darin, dass die Form und Spannung, insbesondere des Deckabschnitts 11, durch den elastisch federnden Seitenabschnitt 15 gegeben ist. Dadurch wird der Deckabschnitt 11, umfassend z. B. eine durchsichtige Polymerfolie, glatt gezogen und horizontal ausgerichtet. Dies ist für eine Beobachtung der hängenden Tropfen, z. B. unter Verwendung eines Mikroskops, von Vorteil, da bei Verwendung einer optisch klaren Folie und eines Objektivs mit hoher Apertur die Zellen 1 unmittelbar im hängenden Tropfen abgebildet und beobachtet werden können. Die optional ebenfalls vorgesehene straffe und horizontale Ausrichtung des Bodenabschnitts 12 hat den Vorteil, dass eine Beleuchtung des Kultivierungsgefäßes von der Unterseite ermöglicht wird. Des Weiteren kann die Flüssigkeit 3, insbesondere Zellen in der Zellsuspension im Bodenabschnitt 12, z. B. mit einem Mikroskop, beobachtet werden.

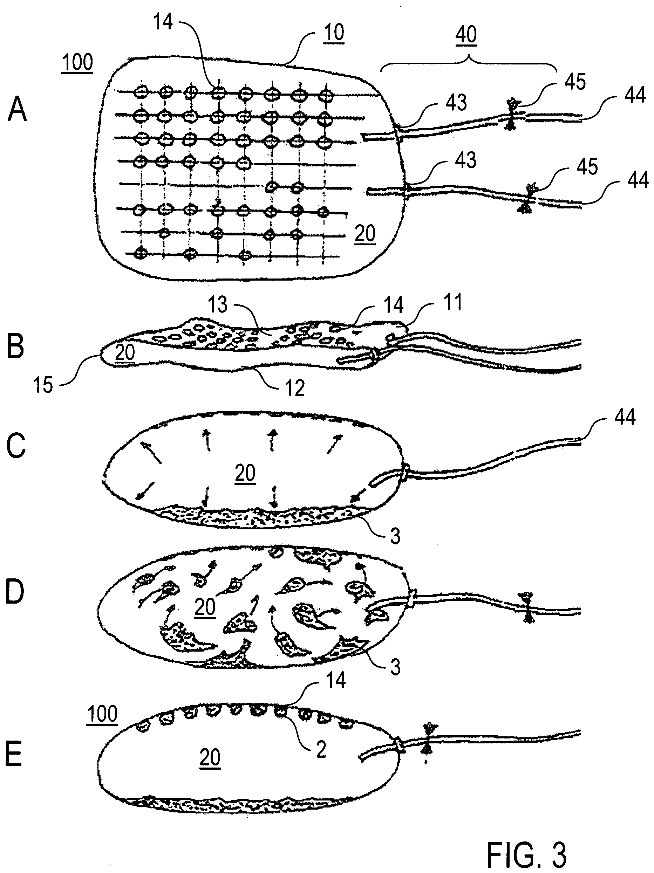

Figur 3 illustriert eine weitere Ausführungsform eines beutelförmigen Kultivierungsgefäßes 100 in verschiedenen Betriebszuständen. Das Kultivierungsgefäß 100 umfasst eine Gefäßwand 10 aus einer Kunststofffolie mit den flächigen Deck- und Bodenabschnitten 11, 12, die an ihren Randbereichen miteinander verbunden sind. Der gekrümmte Übergangsbereich zwischen den Deck- und Bodenabschnitten 11, 12 wird auch als Seitenabschnitt 15 bezeichnet. Die Medien-Einrichtung 40 umfasst zwei Medien-Schnittstellen 43 (Leitungsdurchführungen durch die Gefäßwand 10). Die Medien-Schnittstellen 43 sind für eine gas- und druckdichte Kopplung der Schläuche 44 mit dem Seitenabschnitt 15 ausgelegt. Jede der Medien-Schnittstellen 43 erlaubt die Durchführung eines Schlauchs 44, jeweils mit einem Stellventil 45, in den Innenraum 20 des Kultivierungsgefäßes 100. Die Schläuche führen zu äußeren Reservoiren (nicht dargestellt) für gasförmige oder flüssige Medien. Abweichend von der Illustration können ein einziger Schlauch 44 oder mehr als zwei Schläuche 44 vorgesehen sein.

Die Figuren 3A und 3B zeigen das beutelförmige Kultivierungsgefäß 100 im ungefüllten, sterilen Ausgangszustand in Draufsicht und in geschnittener Perspektivansicht. Der Innenraum 20 ist leer. Die Innenseite des Deckabschnitts 11 bildet die Kultivierungsfläche 13 mit den Halteelementen 14, die mit einem Muster aus Reihen und Spalten angeordnet sind.

Zu Beginn der erfindungsgemäßen Kultivierung biologischer Zellen wird gemäß Figur 3C das Kultivierungsgefäß 100 befüllt. Über einen ersten Schlauch 44 wird die Flüssigkeit 3 zum Bodenabschnitt 12 geleitet, während über einen zweiten Schlauch (nicht dargestellt) ein gasförmiges Medium, z. B. ein Luft-CO₂-Gemisch mit 5% CO₂ zugeführt wird. Unter der Wirkung des gasförmigen Mediums baut sich ein Innendruck im Innenraum 20 auf, so dass das Kultivierungsgefäß 100 den entfalteten Zustand erhält.

Das in Figur 3 illustrierte Verfahren zeigt, dass die hängenden Tropfen im Kultivierungsgefäß 100 nicht zwingend durch eine Deformation der Gefäßwand, sondern durch deren Bewegung als Ganzes realisiert werden kann. Zur Beladung der Kultivierungsfläche 13 wird das Kultivierungsgefäß 100 gemäß Figur 3D geschwenkt. Dabei sind die Schläuche 44 vorzugsweise geschlossen. Die Flüssigkeit 3 verteilt sich im Innenraum 20, so dass sich an den Halteelementen 14 die hängenden Tropfen 2 bilden (Figur 3E). In entfaltetem Zustand gemäß Figur 3E können bei Bedarf Schläuche wieder geöffnet werden, um ergänzend gasförmiges Medium zuzuführen, um die Form des Kultivierungsgefäßes 100 während der Kultivierung zu halten.

Das Kultivierungsgefäß 100 gemäß Figur 3 hat den besonderen Vorteil, dass es für die Kombination der Kultivierung mit einer Kryokonservierung der kultivierten Zellen besonders geeignet ist. Die Gefäßwand kann aus tieftemperaturstabilen Materialien hergestellt sein, so dass das Kultivierungsgefäß 100 mit den hängenden Tropfen während oder nach der Kultivierung ohne weitere Zwischenschritte direkt eingefroren und einer Kühlkonservierung (Lagerung bei tiefen Temperaturen) unterzogen werden kann.

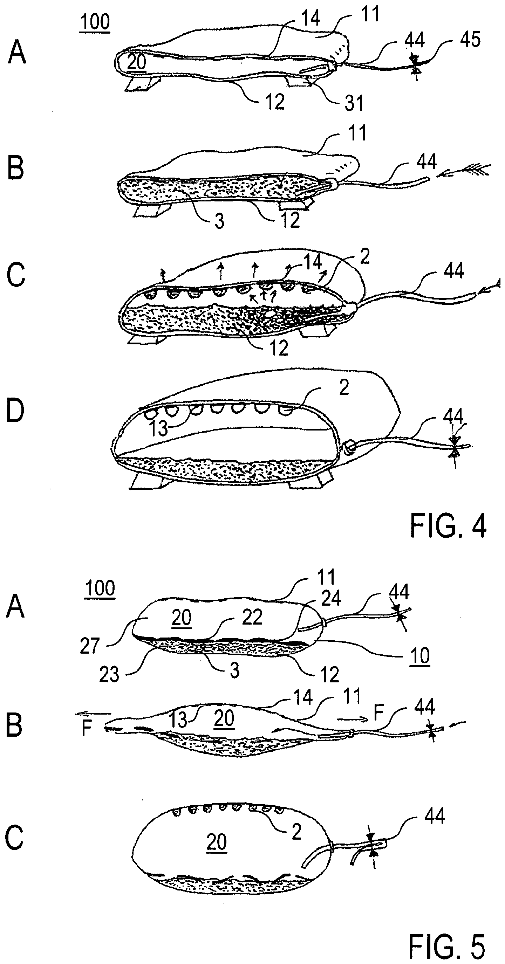

Figur 4 illustriert eine weitere Variante des erfindungsgemäßen Verfahrens, bei dem hängende Tropfen 2 an der Kultivierungsfläche 13 des erfindungsgemäßen Kultivierungsgefäßes 100 gebildet werden. Gemäß Figur 4A ist das Kultivierungsgefäß 100 wie in Figur 3B in Gestalt eines flexiblen Beutels aufgebaut, wobei zusätzlich eine Trägereinrichtung 30 mit Masseelementen 31 am Bodenabschnitt 12 vorgesehen ist. Vor der Beladung mit der Flüssigkeit weist das Kultivierungsgefäß 100 einen sterilen, zusammengefalteten Innenraum 20 auf. Der Schlauch 44 ist mit dem Stellventil 45 geschlossen. Die Masseelemente 31 wirken als stabilisierende Stützfüße auf der Unterseite des Kultivierungsgefäßes 100.

Der Zustand des Kultivierungsgefäßes 100 in Figur 4A entspricht dem komprimierten Zustand, in dem die Deck- und Bodenabschnitte 11, 12 einander angenähert sind. Entsprechend kann die Beschickung des Kultivierungsgefäßes 100 mit der Flüssigkeit 3 und die Beladung der Kultivierungsfläche auf der Innenseite des Deckabschnitts 11 gemäß Figur 4B in einem gemeinsamen Verfahrensschritt erfolgen. Es wird ausschließlich die Flüssigkeit 3 zugeführt, ohne das Kultivierungsgefäß 100 mit einem gasförmigen Medium zu entfalten.

Nach der Benetzung der Kultivierungsfläche gemäß Figur 4B erfolgt in einem weiteren Schritt der Übergang zum entfalteten Zustand des Kultivierungsgefäßes 100 (Figur 4C). Hierzu wird über einen Schlauch 44 ein gasförmiges Medium in das Kultivierungsgefäß 100 gedrückt, so dass sich der Beutel aufwölbt, die Halteelemente 14 von der Flüssigkeit 3 am Boden 12 getrennt und die hängenden Tropfen 2 gebildet werden.

Im prall gefüllten Zustand (Figur 4D) ist der gewünschte Arbeitsabstand des Kultivierungsgefäßes 100 erreicht. In diesem Zustand wird der Schlauch 44 gasdicht geschlossen. Es folgt die Kultivierung der hängenden Tropfen 2 mit den biologischen Zellen entsprechend dem gewünschten Kultivierungsprotokoll.

Figur 5 illustriert eine weitere Ausführungsform des erfindungsgemäßen Kultivierungsgefäßes 100, dessen Gefäßwand 10 mit den Deck- und Bodenabschnitten 11, 12 den Innenraum 20 einschließt. Der Innenraum 20 ist durch eine Zwischenwand 22 in zwei Horizontalkammern 23, 27 unterteilt. Die untere Horizontalkammer 23 ist zur Aufnahme der Flüssigkeit 3 am Bodenabschnitt 12 vorgesehen. Die obere Horizontalkammer 27 ist zur Aufnahme eines gasförmigen Mediums zur Bildung des entfalteten Zustands des Kultivierungsgefäßes 100 vorgesehen. Die Zwischenwand 22 enthält Sollbruchstellen 24, an denen bei Ausübung einer mechanischen Kraft Öffnungen der Zwischenwand 22 gebildet werden können.

Figur 5A zeigt das Kultivierungsgefäß 100 in einem Ausgangszustand. Die untere Horizontalkammer 23 ist mit der Flüssigkeit 3, z. B. einer sterilen Lösung, wie etwa einem Kultivierungsmedium für biologische Zellen, vorbefüllt. Durch die Zwischenwand 22 ist die Flüssigkeit 3 vom übrigen Innenraum 20 getrennt. Die obere Horizontalkammer 27 kann über den Schlauch 44 mit einem gasförmigen Medium befüllt sein, so dass die obere Horizontalkammer 27 teilweise oder vollständig entfaltet ist. Typischerweise erfolgt die Befüllung der oberen Horizontalkammer 27 mit dem gasförmigen Medium erst unmittelbar vor dem Gebrauch des Kultivierungsgefäßes 100.

Für die Kultivierung biologischer Zellen in hängenden Tropfen wird das Kultivierungsgefäß 100 durch außen lateral wirkende Kräfte F in die Breite gezogen, so dass sich die Deck- und Bodenabschnitte 11, 12 einander annähern und gleichzeitig die Sollbruchstellen 24 der Zwischenwand 22 aufreißen (Figur 5B). Über den Schlauch 44 wird eine Zellsuspension mit den zu kultivierenden Zellen in den Innenraum 20 gespült, so dass sich die Zellsuspension mit der Flüssigkeit 3 vermischt. Die Kultivierungsfläche 13 auf der Innenseite des Deckabschnitts 11 wird mit dem Gemisch aus der Flüssigkeit 3 und der Zellsuspension benetzt, so dass sich an den Halteelementen 14 (hydrophile Bereiche) die hängenden Tropfen 2 bilden.

Anschließend wird durch die Zuführung gasförmigen Mediums das Kultivierungsgefäß 100 in den entfalteten Zustand gebracht (Figur 5C), in dem die weitere Kultivierung gemäß dem gewünschten Kultivierungsprotokoll erfolgt. Nach Bildung des entfalteten Zustands werden Schläuche geschlossen (z. B. abgeklemmt) und von äußeren Reservoiren getrennt.

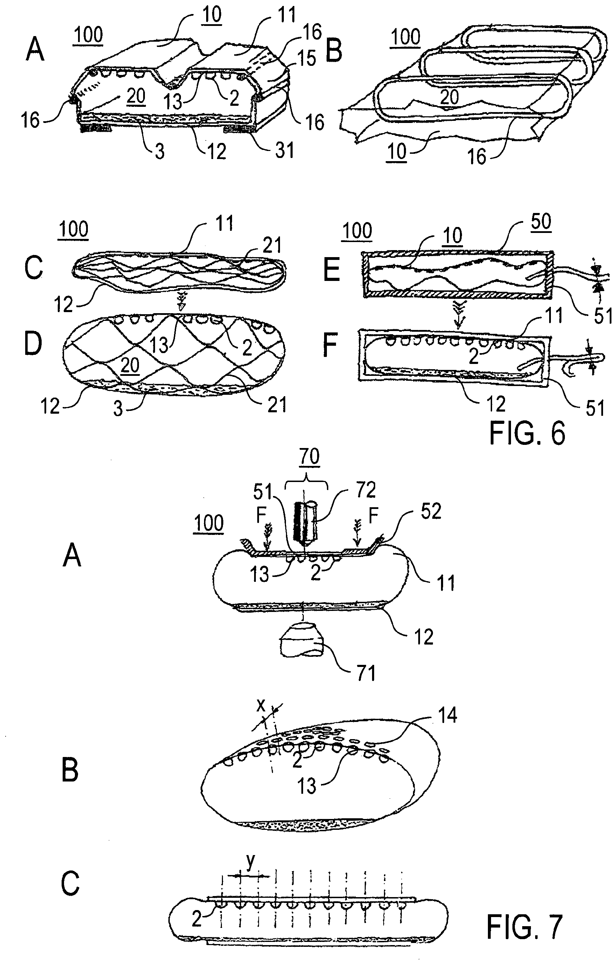

Figur 6 zeigt weitere Varianten des erfindungsgemäßen Kultivierungsgefäßes 100, teils in perspektivischer, geschnittener Teilansicht (Figur 6A), in Phantomansicht (Figur 6B) oder in Schnittansichten (Figur 6C-6F).

Gemäß Figur 6A umfasst die Gefäßwand 10 mit den Deck-, Boden- und Seitenabschnitten 11, 12, 15 zusätzlich Versteifungselemente 16, z. B. in Gestalt von Stäben, die sich in horizontaler und/oder vertikaler Richtung erstrecken. Der Bodenabschnitt 12 umfasst z. B. eine starre Bodenwanne zur Aufnahme der Flüssigkeit 3. Auf der Unterseite der Bodenwanne sind Massenelemente 31 angeordnet. Die Gefäßwand 10 umfasst im Bereich der Deck- und Seitenabschnitte 11, 15 ein Kunststoff-Folienmaterial, das mit den Versteifungselementen 16 aufgespannt wird. Die Versteifungselemente 16 bestehen aus einem elastisch deformierbaren Kunststoffmaterial, z. B. Silikon, das bei Deformation elastische Rückstellkräfte erzeugt. Durch die Gestalt und Anordnung der Versteifungselemente 16 kann in Abhängigkeit von den konkreten Anwendungsbedingungen die Form des Innenraums 20 bestimmt werden. Zur Bildung des komprimierten Zustands des Kultivierungsgefäßes 100 wird der Deckabschnitt 11 entgegen den elastischen Rückstellkräften durch die Versteifungselemente 16 an den Bodenabschnitt 12 angenähert, bis die Kultivierungsfläche 13 auf der Innenseite des Deckabschnitts 11 mit der Flüssigkeit 3 benetzt wird und die hängenden Tropfen 2 gebildet werden.

Gemäß Figur 6B sind die Versteifungselemente 16 in Gestalt von Ringen aus einem elastischen Material gebildet. Die Ringe sind auf der Innenseite der Gefäßwand 10 befestigt, z. B. angeschweißt. Durch die Form und Anordnung der Versteifungselemente 16 wird die Gestalt des Kultivierungsgefäßes 100 im entfalteten Zustand bestimmt. Zur Kompression des Kultivierungsgefäßes 100 wird eine äußere Kraft zur Deformation der Gefäßwand 10 und insbesondere der Versteifungselemente 16 ausgeübt.

In den Figuren 6C und 6D ist eine Ausführungsform des erfindungsgemäßen Kultivierungsgefäßes 100 gezeigt, bei dem ein faltbarer Innenträger 21 im Innenraum 20 vorgesehen ist. Der faltbare Innenträger 21 besteht z. B. aus einem elastisch deformierbaren Drahtgeflecht aus einem biokompatiblen Metall oder aus einem elastisch deformierbaren Kunststoffgeflecht. Im komprimierten Zustand des Kultivierungsgefäßes 100 (Figur 6C) ist der Innenträger 21 zusammengedrückt, so dass, wie oben beschrieben, die Kultivierungsfläche auf der Innenseite des Deckabschnitts 11 mit der Flüssigkeit (nicht dargestellt) am Bodenabschnitt 12 benetzt werden kann. Nach Entfaltung des Innenträgers 21 (Figur 6D) wird der gewünschte Arbeitsabstand zwischen den Deck- und Bodenabschnitten 11, 12 eingestellt. Teile der Flüssigkeit haben sich an der Kultivierungsfläche 13 in Gestalt von hängenden Tropfen 2 gesammelt.

Gemäß den Figuren 6E und 6F ist das erfindungsgemäße Kultivierungsgefäß 100, z. B. gemäß Figur 3, mit einer äußeren Aufnahmeeinrichtung 50 ausgestattet. Die Aufnahmeeinrichtung 50 hat die Gestalt eines quaderförmigen Kastens mit ebenen Kastenwänden 51. Der Kasten ist zur Aufnahme der Gefäßwand 10 im komprimierten (Figur 6E) und im entfalteten Zustand (Figur 6F) eingerichtet. Die Kastenwände bilden im entfalteten Zustand Anlageflächen für die Gefäßwand 10, so dass insbesondere der Deckabschnitt 11 mit den hängenden Tropfen 2 im entfalteten Zustand eine ebene, horizontale Ausrichtung erhält. Die Innendimension des Kastens ist so gewählt, dass bei der Beaufschlagung des Kultivierungsgefäßes mit einem Innendruck und Anlegen der Deck- und Bodenabschnitte 11, 12 an den oberen und unten Kastenwänden 51 der gewünschte Arbeitsabstand zur Aufnahme der hängenden Tropfen 2 gebildet wird.

Vorteilhafterweise kann mindestens eine der oberen und unteren Kastenwände 51 aus einem optisch klaren, transparenten Material hergestellt sein. In diesem Fall ist eine Beobachtung der hängenden Tropfen 2, insbesondere mit einem Mikroskop, auch bei einer Kultivierung möglich, während der das Kultivierungsgefäß 100 in der Aufnahmeeinrichtung angeordnet ist.

Figur 7 illustriert weitere Einzelheiten der Erfindung, die für die mikroskopische Beobachtung der Kultivierung und/oder eine Automatisierung der Kultivierung von Bedeutung sind. In Figur 7A ist das Kultivierungsgefäß 100, z. B. gemäß Figur 3, in entfaltetem Zustand gezeigt. Die hängenden Tropfen 2 befinden sich auf der Kultivierungsfläche 13 an der Innenseite des Deckabschnitts 11 mit dem gewünschten Arbeitsabstand vom Bodenabschnitt 12. Eine Beobachtungseinrichtung 70, wie z. B. ein Mikroskop (nicht vollständig gezeigt), umfasst unterhalb des Bodenabschnitts 12 eine Beleuchtungseinrichtung 71 und oberhalb des Deckabschnitts 11 ein Objektiv 72. Auf der Außenseite des Deckabschnitts 11 ist eine planare Platte 52 mit einem optisch klaren, transparenten Fenster 51 vorgesehen. Die Platte 52 weist an ihren äußeren Rändern abgeschrägte Randabschnitte auf. Die Randabschnitte stabilisieren vorteilhafterweise die Lage der Platte 52 auf dem Deckabschnitt 11. Bei Ausübung von äußeren Kräften F wird die Platte 52 teilweise in das Kultivierungsgefäß 100 eingedrückt, so dass eine seitliche Verschiebung unterbunden wird. Mit der Platte 52 wird die Planarität der Gefäßwand im Deckabschnitt 11 verbessert. Durch das Fenster 51 können die hängenden Tropfen 2 mit der Beobachtungseinrichtung 70 optisch erfasst werden. Alternativ kann die Platte 52 z. B. Teil einer Aufnahmeeinrichtung 50 in Figur 6E sein.

Wenn die Halteelemente 14 der Kultivierungsfläche 13 vorbestimmte, bekannte Positionen aufweisen und ein bestimmtes geometrisches, vorzugsweise regelmäßiges Muster bilden, so ergeben sich Vorteile für eine Automatisierung der Beobachtung mit der Beobachtungseinrichtung 70. Wenn die Halteelemente 14 z. B. in geraden Reihen und Spalten mit Reihenabständen x und Spaltenabständen y (siehe Figuren 7B und 7C) angeordnet sind, können mit dem Objektiv 72 (siehe Figur 7A) die einzelnen hängenden Tropfen 2 vereinfacht angefahren werden. Mit der Beobachtungseinrichtung 70 können dann z. B. Wachstums- und/oder Differenzierungsprozesse in ihrem zeitlichen Verlauf dokumentiert werden. Figur 7B zeigt, dass die mikroskopische Beobachtung der hängenden Tropfen 2 auch ohne die Platte 52 möglich ist. Die Fokussierung bei der mikroskopischen Abbildung wird jedoch verbessert, wenn die Platte (siehe Figuren 7A, 7C) verwendet wird.

Gemäß einer weiteren Variante der Erfindung kann auf der Außenseite der Gefäßwand des erfindungsgemäßen Kultivierungsgefäßes oder auf einer auf die Gefäßwand aufgesetzten Platte eine Anordnung optischer Elemente, z. B. eine LinsenAnordnung, vorgesehen sein, um die optische Beobachtung der hängenden Tropfen zu vereinfachen. Die Bereitstellung z. B. von Linsen-Anordnungen umfasst eine Vielzahl von Linsen, die jeweils auf einen der hängenden Tropfen produziert sind, ermöglicht die Integration von linsenfreien Mikroskop-Arrays und vereinfacht ein permanentes Überwachen der Kultivierung in den hängenden Tropfen.

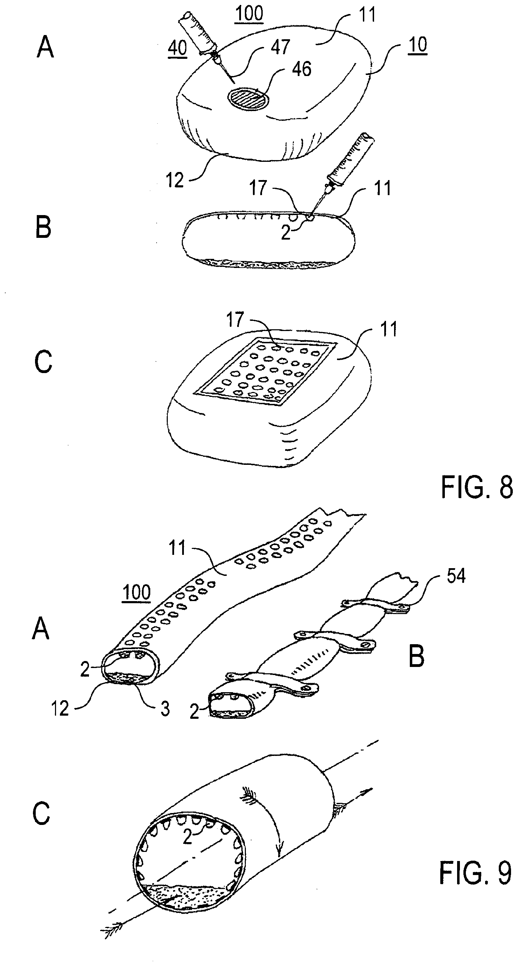

Ein wichtiges Merkmal des erfindungsgemäßen Kultivierungsgefäßes besteht darin, dass der Innenraum 20 (siehe z. B. Figur 1) gegenüber der Umgebung geschlossen ist. Um dennoch einen Zugriff auf den Innenraum 20 zu ermöglichen, ist das Kultivierungsgefäß 100 vorzugsweise mit der Medien-Einrichtung 40 ausgestattet, die gemäß den Ausführungsformen in den Figuren 1 und 3 für ein reversibles Öffnen und Schließen des Kultivierungsgefäßes 100 ausgelegt ist. Figur 8 zeigt weitere Varianten einer Medieneinrichtung 40, die für ein reversibles oder irreversibles Öffnen des Kultivierungsgefäßes 100 ausgelegt ist.

Gemäß Figur 8A enthält die Gefäßwand 10, insbesondere im Deckabschnitt 11, ein Septum 46, über das flüssige oder gasförmige Medien mittels einer Kanüle 47 eingebracht oder entnommen werden können. Beispielsweise kann mit der Spritzenkanüle einer Spritze das Septum 46 durchstochen werden, um Flüssigkeit zum Bodenabschnitt 12 zuzuführen. Alternativ ist ein Zugriff auf die hängenden Tropfen 2 durch entsprechende Fensterabschnitte 17 im Deckabschnitt 11 möglich, wie in den Figuren 8B und 8C gezeigt ist. Es kann insbesondere gemäß Figur 8C in dem Deckabschnitt 11 ein Fensterabschnitt 17 mit Sollbruchstellen integriert sein, deren Positionen passend zu den Positionen der Halteelemente auf der Kultivierungsfläche auf der Innenseite des Deckabschnitts 11 gewählt sind. Die Sollbruchstellen können mit einem Werkzeug, wie z. B. einer Kanüle, durchbrochen werden, um den hängenden Tropfen Zusatzsubstanzen zuzuführen und/oder Proben aus den hängenden Tropfen zu entnehmen.

Die Form des erfindungsgemäßen Kultivierungsgefäßes 100 ist nicht auf die abgeflachte Kissen- oder Quaderform beschränkt, wie sie z. B. oben unter Bezug auf Figur 1 oder 3 beschrieben ist. Alternativ kann das Kultivierungsgefäß eine Schlauchform aufweisen, wie schematisch in den perspektivischen, geschnittenen Teilansichten von Figur 9 gezeigt ist. Gemäß Figur 9A umfasst das Kultivierungsgefäß 100 einen abgeflachten, deformierbaren, an seinen Enden verschließbaren Schlauch, z. B. aus Kunststoff, der sich in einer Längsrichtung erstreckt, und auf einer ersten Längsseite (Oberseite) den Deckabschnitt 11 zur Aufnahme der hängenden Tropfen 2 und an einer zweiten Längsseite (Unterseite) den Bodenabschnitt 12 zur Aufnahme der Flüssigkeit 3 aufweist. Gemäß Figur 9B kann der Schlauch entlang der Längsrichtung mit Klammern 54 unterteilt werden. Die Klammern 54 erfüllen eine Doppelfunktion erstens zur Stabilisierung der Lage des Schlauches auf einer Unterlage, z. B. einem Labortisch, und zweitens zur Bildung getrennter Bereiche für die hängenden Tropfen 2.

Die Beladung der Kultivierungsfläche 13 auf der Innenseite des Deckabschnitts 11 erfolgt durch eine Deformation des Schlauches, wie oben z. B. unter Bezug auf die Figuren 1 oder 2 beschrieben wurde. Alternativ kann, wie in Figur 9C gezeigt ist, eine Bewegung der Gefäßwand 10 zur Benetzung von Halteelementen 14 mit der Flüssigkeit 3 vorgesehen sein. Durch eine Drehung um die Längsachse des Schlauches (siehe gekrümmter Pfeil) fließt die Flüssigkeit 3 über die Halteelemente 14, wo bei weiterer Drehung Tropfen hängen bleiben.

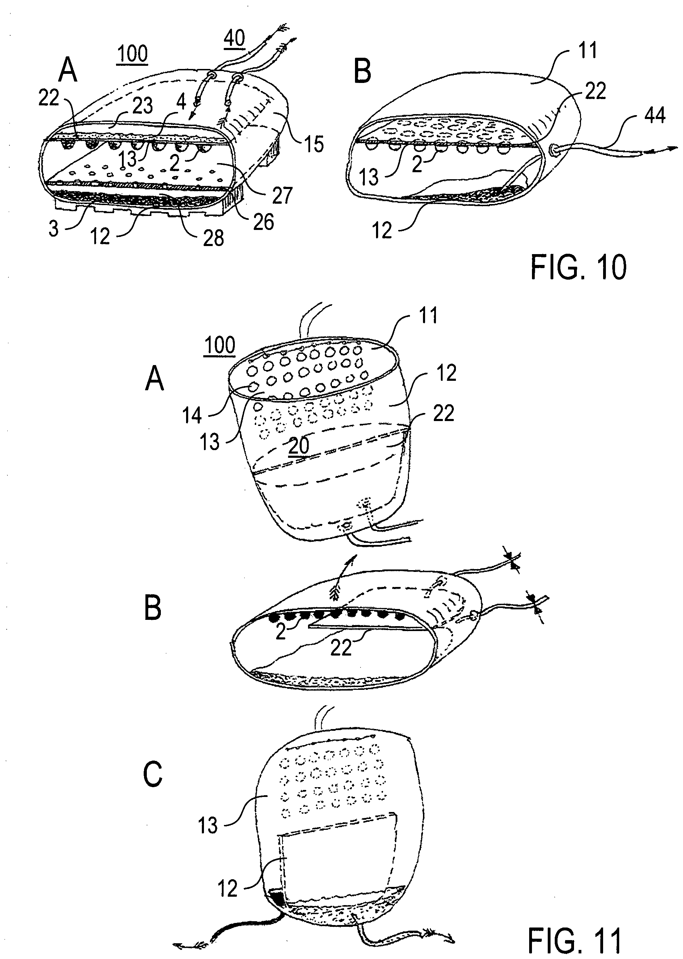

In Figur 10 sind weitere Einzelheiten von erfindungsgemäßen Kultivierungsgefäßen 100 gezeigt, bei denen zusätzliche Freiheitsgrade zur Beeinflussung der hängenden Tropfen bestehen. Gemäß Figur 10A ist der Innenraum des Kultivierungsgefä-βes 100 durch zwei Zwischenwände 22, 26 in drei Horizontalkammern 23, 27, 28 unterteilt. Die obere Horizontalkammer 23 wird über eine Medien-Einrichtung 40 mit einer Flüssigkeit 4 befüllt. Die mittlere Horizontalkammer 27 wird mit einem gasförmigen Medium gefüllt, und enthält temporär Flüssigkeit aus der unteren Horizontalkammer 28. Die untere Horizontalkammer 28 enthält eine Flüssigkeit 3. Die Gestalt des Kultivierungsgefäßes 100 im entfalteten Zustand wird z. B. durch eine Elastizität des Seitenabschnitts 15 bestimmt.

Die Kultivierungsfläche 13 ist an der Unterseite der oberen Zwischenwand 22 vorgesehen. Die obere Zwischenwand 22 erlaubt eine molekulare Diffusion von Substanzen aus der Flüssigkeit 4 in die hängenden Tropfen 2. Die diffundierenden Substanzen umfassen z. B. Ionen, Differenzierungsfaktoren, Hormone oder dergleichen. Die untere Zwischenwand 26 umfasst eine perforierte Folie, welche die Flüssigkeit 3 in der unteren Horizontalkammer 28 von der Horizontalkammer 27 trennt. Die untere Zwischenwand 26 bietet einen Schutz der hängenden Tropfen 2 gegen unerwünschte Flüssigkeitsbewegungen im Bodenabschnitt 12. Wenn durch eine Bewegung des Kultivierungsgefäßes 100 Flüssigkeitsspritzer nach oben fliegen, so werden diese von der unteren Zwischenwand 22 aufgefangen.

Das erfindungsgemäße Kultivierungsverfahren erfolgt bei Gebrauch der Ausführungsform gemäß Figur 10A derart, dass zunächst die Flüssigkeit 3 am Bodenabschnitt 12 bereitgestellt wird. Des Weiteren wird die obere Horizontalkammer 23 mit der Flüssigkeit 4 gefüllt. Die Benetzung der Kultivierungsfläche 13 an der Unterseite der oberen Zwischenwand 22 erfolgt, wie oben beschrieben wurde, durch eine Deformation des Kultivierungsgefäßes 100. Durch eine äußere Kraft werden der Deck- und der Bodenabschnitt 11, 12 zueinander gedrückt. Die Flüssigkeit 3 dringt durch die untere Zwischenwand 26 in die mittlere Horizontalkammer 27, wo die Kultivierungsfläche 13 benetzt wird. Die Flüssigkeit 3 sammelt sich an den Halteelementen der Kultivierungsfläche 13, so dass nach der anschließenden Entfaltung des Kultivierungsgefäßes 100 die hängenden Tropfen 2 gebildet werden. Mittels Diffusion erfolgt während der Kultivierung der Zusatz von Substanzen aus der Flüssigkeit 4 in die hängenden Tropfen 2. Beispielsweise durch Differenzierungsfaktoren wird die Differenzierung der Zellen in den hängenden Tropfen 2 beeinflusst.

Ersatzweise kann das Kultivierungsgefäß 100 an den Deckabschnitt 11 angrenzend eine einzige Zwischenwand 22 aufweisen. An der Unterseite der Zwischenwand 22 ist die Kultivierungsfläche 13 zur Aufnahme der hängenden Tropfen 2 gebildet. Figur 10B zeigt den Zustand der hängenden Tropfen 2 an der Zwischenwand 22 am Ende eines Kultivierungsvorganges. Wenn die kultivierten Zellen gesammelt werden sollen, wird zunächst über den Schlauch 44 Restflüssigkeit vom Bodenabschnitt 12 entfernt. Bei Bedarf können über den Schlauch 44 Puffer- oder Salzlösungen für Spülvorgänge zu- und abgeführt werden. Anschließend werden die hängenden Tropfen 2 mit den daran befindlichen Zellen oder Zellaggregaten von der Zwischenwand 22 abgeschüttelt, so dass in den Bodenabschnitt 12 fallen. Dort werden sie mit einer Pufferlösung abgespült, die über den Schlauch 44 zu- und abgeführt wird. Die gesammelten Zellen oder Zellaggregate können ebenfalls über den Schlauch 44 aus dem Kultivierungsgefäß 100 entfernt werden.

Wenn sich eine Zwischenwand 22 nur teilweise im Innenraum 20 des erfindungsgemäßen Kultivierungsgefäßes 100 erstreckt, kann die Sammlung (Ernte) der kultivierten Zellen aus den hängenden Tropfen modifiziert werden, wie schematisch in Figur 11 gezeigt ist. Figur 11A zeigt in schematischer, geschnittener Perspektivansicht ein beutelförmiges Kultivierungsgefäß 100 mit dem Deckabschnitt 11 und dem Bodenabschnitt 12 in einer vertikalen Ausrichtung. Die Zwischenwand 22 erstreckt sich über einen Teil, z. B. die Hälfte, des Innenraums 20. Halteelemente 14 sind an der Kultivierungsfläche 13 nur in dem Bereich vorgesehen, in dem sich die Zwischenwand 22 nicht erstreckt.

Die Beladung der Kultivierungsfläche 13 und die Kultivierung von Zellen in hängenden Tropfen erfolgt, wie oben z. B. unter Bezug auf die Figuren 1 oder 2 beschrieben wurde. Nach Beendigung der Kultivierung (Figur 11B) wird das Kultivierungsgefäß 100 gedreht (siehe Pfeil). Zur Sammlung (Ernte) der Zellen oder Zellaggregate können diese von der Kultivierungsfläche 13 gezielt in eine Teilkammer auf einer Seite der Zwischenwand 22 gespült werden können, während die ursprünglich vorhandene Zellsuspension auf der gegenüberliegenden Seite der Zwischenwand 22 bleibt (siehe Figur 11C).

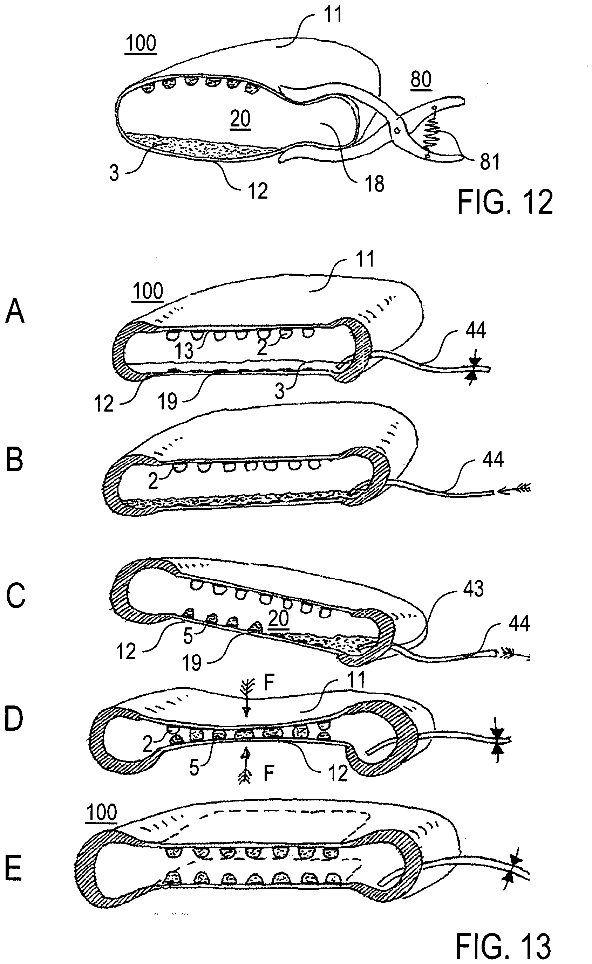

In Figur 12 ist schematisch eine Klemmeinrichtung 80 gezeigt, mit der das Volumen des Innenraums 20 eines erfindungsgemäßen Kultivierungsgefäßes 100 beeinflusst werden kann. Wenn das Kultivierungsgefäß 100 zur Bildung des entfalteten Zustands, z. B. gemäß Figur 3C, mit einem Innendruck beaufschlagt wurde, können während der über Tage oder Wochen dauernden Kultivierung Gasverluste auftreten. Im Ergebnis könnte das Kultivierungsgefäß 100 in unerwünschter Weise kollabieren. Um dies zu vermeiden, wird mit der Klemmeinrichtung 80, die z. B. einen Federantrieb 81 enthält, in einem Quetschabschnitt 18 der Gefäßwand 10 ein konstanter Druck auf das Kultivierungsgefäß 100 ausgeübt. Dadurch wird die straffe Form des Beutels und der ausreichende Abstand der hängenden Tropfen 2 von der Flüssigkeit 3 am Bodenabschnitt 12 auch bei Gasverlusten garantiert. Alternativ zu der Variante in Figur 12 kann der konstante Druck durch einen Wickelabschnitt an einer Seite des Kultivierungsgefäßes, einen separaten Beutelteil, an dem eine Klemmeinrichtung angesetzt ist, oder einen über einen Schlauch angeschlossenen Zusatzbeutel (nicht dargestellt) aufrechterhalten werden.

Figur 13 illustriert beispielhaft einen Verfahrensablauf zur Kryokonservierung von Zellen in hängenden Tropfen. Die Kryokonservierung erfolgt z. B. nach der Kultivierung in den hängenden Tropfen, um die gewonnen Zellen dauerhaft zu lagern. Es wird ein Kultivierungsgefäß 100 verwendet, das im Wesentlichen wie in Figur 2 aufgebaut ist. Zusätzlich weist das Kultivierungsgefäß 100 auf der Innenseite des Bodenabschnitts 12 hydrophile Oberflächenbereiche 19 auf, die durch hydrophobe Oberflächenbereiche voneinander getrennt sind. Die hydrophilen Oberflächenbereiche 19 sind passend zur geometrischen Anordnung der Halteelemente 14 an der Kultivierungsfläche 13 am Deckabschnitt 11 positioniert.

Figur 13A zeigt das Kultivierungsgefäß 100 im entfalteten Zustand, in dem die hängenden Tropfen 2 an der Kultivierungsfläche 13 angeordnet sind und der Bodenabschnitt 12 mit einer Flüssigkeit 3 bedeckt ist. Zur Vorbereitung der Kryokonservierung der Tropfen 2 wird der Flüssigkeit 3 über den Schlauch 44 ein Kryoprotektivum, z. B. DMSO, zugesetzt, oder die Flüssigkeit 3 wird durch eine Lösung eines Kryoprotektivums ersetzt (Figur 13B). Anschließend wird das Kultivierungsgefäß 100 gekippt, so dass die Flüssigkeit im Innenraum 20 zur Medien-Schnittstelle 43 fließt und über den Schlauch 44 abfließen kann. Dabei bleiben auf den hydrophilen Oberflächenbereichen 19 des Bodenabschnitts 12 Tropfen 5 mit dem Kryoprotektivum zurück (Figur 13C).

Anschließend wird das Kultivierungsgefäß 100 in den komprimierten Zustand überführt (Figur 13D). Die Deck- und Bodenabschnitte 11, 12 werden unter der Einwirkung äußerer Kräfte F zueinander geführt, bis die Tropfen 2, in denen die biologischen Zellen kultiviert wurden, und die Tropfen 5 miteinander verschmelzen. Da die Tropfen 2, 5 auf beiden Seiten einander exakt gegenüberliegen und die lateralen Abstände zwischen den Tropfen ausreichend groß sind, bleiben die Zellen in den Tropfen an reproduzierbaren Positionen.

Die Kryokonservierung kann im komprimierten Zustand (Figur 13D) oder im anschließend wieder entfalteten Zustand (Figur 13E) des Kultivierungsgefäßes 100 erfolgen. Hierzu wird das Kultivierungsgefäß 100 in eine Umgebung verminderter Temperatur, z. B. in flüssigen Stickstoff oder in den kalten Dampf über flüssigem Stickstoff in einen Stickstofftank gebracht. Im gefrorenen Zustand können die Wandbereiche, welche die Tropfen tragen, von den übrigen Teilen des Kultivierungsgefäßes 100 getrennt werden.

Die Gefäßwand des erfindungsgemäßen Kultivierungsgefäßes 100 kann mit Temperaturleitelementen, wie z. B. einer Metallbeschichtung, ausgestattet sein, um die Abkühlung bei der Beaufschlagung mit dem Kühlmittel zu beschleunigen. Des Weiteren kann das Kultivierungsgefäß 100 mit Heizelementen, wie z. B. Widerstandsheizelementen, ausgestattet sein, die in die Gefäßwand integriert sind. Die Heizelemente können ein schnelles und gleichmäßiges Auftauen nach Beendigung der Kühlkonservierung unterstützen.

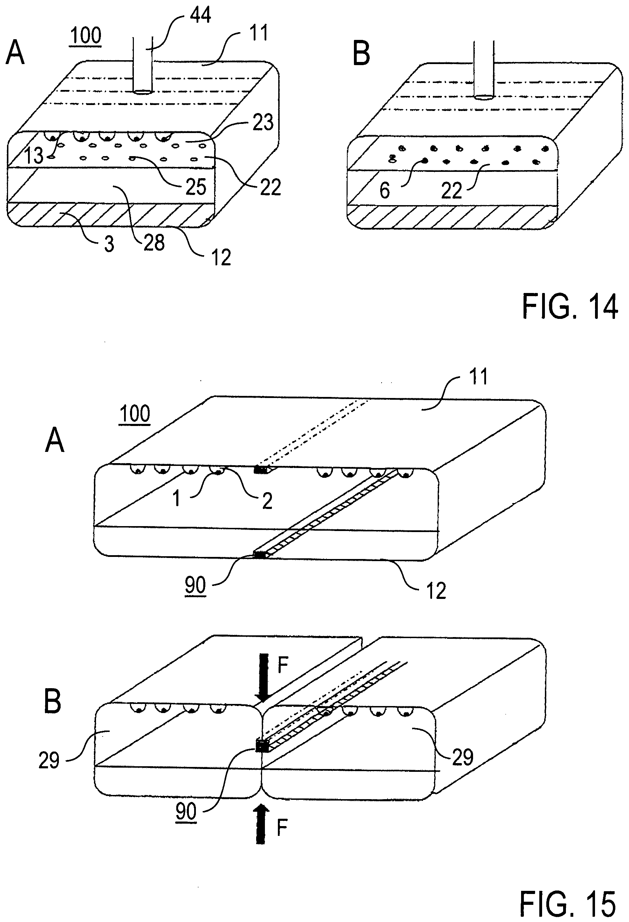

Figur 14 zeigt eine weitere Variante eines erfindungsgemäßen Kultivierungsgefäßes 100, dessen Innenraum 20 durch eine Zwischenwand 22 in eine obere (23) und eine untere (28) Horizontalkammer geteilt ist. Die Zwischenwand 22 ist eine Kunststofffolie, die Poren 25 einer definierten Größe (> 100 pm) aufweist.

Die Bildung der hängenden Tropfen 2 und deren Kultivierung erfolgt, wie oben beschrieben wurde. Im komprimierten Zustand des Kultivierungsgefäßes 100 durchdringt die Flüssigkeit 3 vom Bodenabschnitt 12 die Zwischenwand 22, so dass die Kultivierungsfläche 13 benetzt wird und die hängenden Tropfen 2 an den Halteelementen gesammelt werden. Bei der Ernte der kultivierten Zellen kann die Aufgabe darin bestehen, Zellaggregate von Einzelzellen zu trennen. Hierzu wird über den Schlauch 44 ein Kultivierungsmedium oder eine Pufferlösung zugeführt, um die hängenden Tropfen 2 von der Kultivierungsfläche 13 abzuspülen. Die Flüssigkeit und die Einzelzellen können die Poren in der Zwischenwand 22 passieren, während die Zellaggregate 6 an der Zwischenwand 22 immobilisiert werden (Figur 14B). Anschließend kann die Zwischenwand 22 an vorbestimmten Sollbruchstellen aus dem Kultivierungsgefäß 100 gelöst werden, um die aufgefangenen Zellaggregate weiterführenden Untersuchungen zuzuführen.

Figur 15 illustriert eine weitere Ausführungsform des erfindungsgemäßen Kultivierungsgefäßes 100, bei dem die Deck- und Bodenabschnitte 11, 12 mit linienförmigen Koppelelementen 90 ausgestattet sind. Die Koppelelemente 90 umfassen Kompositmaterialien, wie Polyethylen, Polypropylen auf den Innenseiten der Deck- und Bodenabschnitte 11, 12, die in Reaktion auf einen äußeren Druck miteinander verbunden werden. Die Verwendung des Kultivierungsgefäßes 100 gemäß Figur 15 erfolgt derart, dass zunächst die hängenden Tropfen 2 mit den biologischen Zellen 1 gebildet werden, wie oben beschrieben wurde. Wenn nach einer bestimmten Kultivierungsdauer ein Teil des Kultivierungsgefäßes 100 abgetrennt werden soll, z. B. um eine Kryokonservierung oder eine weitere Differenzierung durchzuführen oder eine gesonderte Lagerung vorzunehmen, werden auf die Koppelelemente 90 äußere Kräfte F (Figur 15B) ausgeübt, so dass die Koppelelemente 90 miteinander verschmelzen. Dadurch werden zwei Vertikalkammern 29 gebildet, die voneinander trennbar sind.

Bei der in den Figuren 16 bis 20 gezeigten Variante des erfindungsgemäßen Kultivierungsgefäßes 100 umfassen die Halteelemente 14 hydrophile Stufenelemente in Gestalt von umlaufenden Ringen 14.1, die an der Kultivierungsfläche 13 positioniert sind und die jeweils eine hydrophilen Oberflächenabschnitt 14.2 der Kultivierungsfläche 13 einschließen. Die Ringe 14.1 sind mit einer Höhe von z. B. 500 pm bis 1 mm und einem Durchmesser von z. B. 5 bis 20 mm aus Kunststoff hergestellt. Die Gefäßwand des Kultivierungsgefäßes 100 ist im Bereich des Seitenabschnitts 15 mit einer Drehhalterung 60 und einem Schlauch 44 ausgestattet. Die Drehhalterung 60 umfasst zwei seitlich abstehende Zapfen 61, mit denen das Kultivierungsgefäß 100 in einen Träger (nicht dargestellt) eingehängt und gedreht werden kann. Über den Schlauch 44 können Medien in das Innere des Kultivierungsgefäßes 100, insbesondere in den Bodenabschnitt 12, geleitet werden.

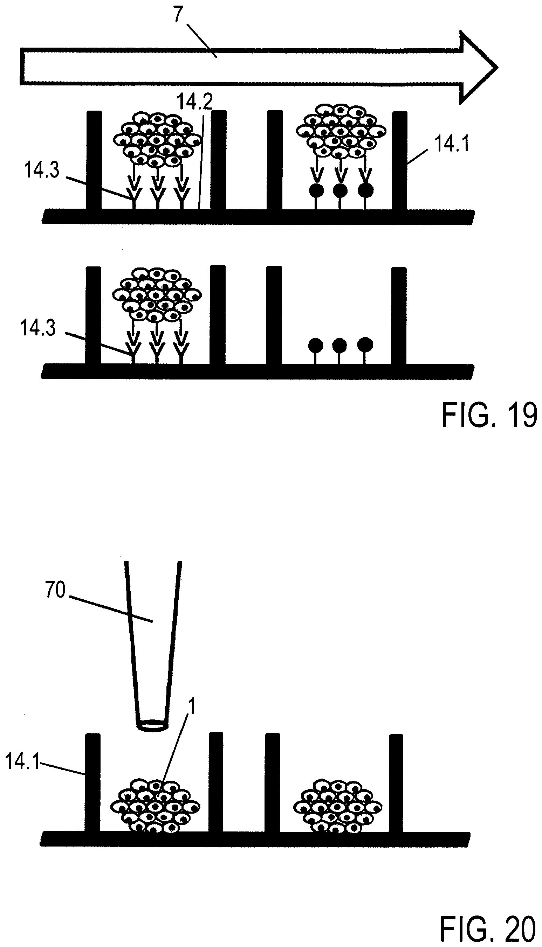

Die Kultivierung im Kultivierungsgefäß 100 gemäß Figur 16 erfolgt, indem zunächst die Halteelemente 14 mit hängenden Tropfen 2 jeweils mit Zellen beladen werden, wie oben beschrieben wurde. Im Ergebnis der Kultivierung vermehren sich die Zellen zu Zellaggregaten 1. Eine Draufsicht auf einen Ausschnitt der Kultivierungsfläche 13 ist in Figur 17 gezeigt. Die Tropfen 2 mit den Zellaggregaten 1 sind in den hydrophilen Oberflächenabschnitten 14.2 positioniert, die durch die Ringe 14.1 begrenzt sind. Die hydrophilen Oberflächenabschnitt 14.2 können zusätzlich mit spezifischen, z. B. fluoreszenzmarkierten Antikörpern besetzt sein (siehe Figur 19) .

Für eine weitere, adhärente Kultivierung wird über den Schlauch 44 die Flüssigkeit vom Bodenabschnitt 12 des Kultivierungsgefäßes 100 abgesaugt. Anschließend wird das Kultivierungsgefäß 100 mit Hilfe der Drehhalterung 60 um 180° gedreht, wie in Figur 18 gezeigt ist. Nun setzen sich die Zellaggregate 1 ab und können innerhalb der von den Ringen 14.1 begrenzten Flächen auf der Kultivierungsfläche adhärieren. Nach der Adhäsion kann über den Schlauch 44 Kulturmedium eingefüllt werden, so dass eine anschließende adhärente Kultivierung der Zellaggregate 1 in den Tropfen 2 möglich ist. Wenn die Oberflächenabschnitte 14.2 mit spezifischen Antikörpern 14.3 beladen sind, kommt es zu einer Antigen-Antikörper-Bindung mit den Zellaggregaten 1 (Figur 19). Dies kann zum einen zur Zellseparation genutzt werden, indem die nicht gebundenen Zellaggregate mit einem Flüssigkeitsstrom 7 durch den Schlauch weggespült werden (siehe Figur 19, unten rechts). Wenn die Antikörper zusätzlich mit Fluoreszenzmarkern versehen sind, kann im Kultivierungsgefäß eine Detektion von spezifischen Zellen erfolgen, z. B. mittels einer Fluoreszenzmessung. Des Weiteren können die Zellaggregate 1, die räumlich voneinander getrennt innerhalb der Ringe 14.1 in eigenen Kavitäten vorliegen, für Screenings genutzt werden, indem in die Kavitäten mit Hilfe einer Pipette 70 manuell oder automatisiert unterschiedliche Substanzen hinzugefügt werden (Figur 20).

Die in der vorstehenden Beschreibung, den Zeichnungen und den Ansprüchen offenbarten Merkmale der Erfindung können sowohl einzeln als auch in Kombination für die Verwirklichung der Erfindung in ihren verschiedenen Ausgestaltungen von Bedeutung sein, jedoch wird die Erfindung durch den Wortlaut der Ansprüche widergegeben.

## Patentansprüche

1. Kultivierungsgefäß (100), das zur Kultivierung biologischer Zellen (1) in hängenden Tropfen (2) eingerichtet ist, umfassend
- eine Gefäßwand (10), die einen Deckabschnitt (11) und einen Bodenabschnitt (12) aufweist, wobei
- der Deckabschnitt (11) zur Bereitstellung einer Kultivierungsfläche (13) eingerichtet ist und der Bodenabschnitt (12) zur Aufnahme einer Flüssigkeit (3) eingerichtet und im Innenraum des Kultivierungsgefäßes (100) zur Kultivierungsfläche (13) gegenüberliegend angeordnet ist,
- die Gefäßwand (10) einen Innenraum (20) des Kultivierungsgefäßes allseits einschließt,
- die Kultivierungsfläche (13) Halteelemente (14) aufweist, die zur Positionierung der Tropfen (2) eingerichtet sind, wobei die Kultivierungsfläche (13) die Tropfen (2) im Innenraum (20) frei hängend aufnehmen kann, und
- die Gefäßwand (10) so beweglich ist, dass mittels einer Bewegung von Teilen der Gefäßwand relativ zueinander die Halteelemente (14) mit der Flüssigkeit (3) vom Bodenabschnitt (12) benetzt werden können,
**dadurch gekennzeichnet, dass**
- die Gefäßwand (10) deformierbar ist und der Deckabschnitt (11) und der Bodenabschnitt (12) relativ zueinander beweglich sind, so dass deren gegenseitiger Abstand bei einer Deformation der Gefäßwand (10) verringert und die Halteelemente (14) an den Bodenabschnitt (12) angenähert werden können.

2. Kultivierungsgefäß gemäß Anspruch 1, bei dem die Halteelemente (14) umfassen
- hydrophile Oberflächenbereiche der Kultivierungsfläche (13), die durch hydrophobe Oberflächenbereiche voneinander getrennt sind.

3. Kultivierungsgefäß gemäß Anspruch 2, bei dem
- die hydrophilen Oberflächenbereiche ein regelmäßiges Muster bilden,
- die hydrophilen Oberflächenbereiche durch eine Oberflächenfunktionalisierung der Kultivierungsfläche (13) gebildet werden, und/oder
- die hydrophilen Oberflächenbereiche durch Stufenelemente an der Kultivierungsfläche (13) gebildet werden.

4. Kultivierungsgefäß gemäß einem der vorhergehenden Ansprüche, bei dem die Gefäßwand (10) mindestens eines der Merkmale umfasst
- die Gefäßwand (10) ist aus einem biegsamen Folienmaterial hergestellt, wobei der Abstand zwischen den Deck- und Bodenabschnitten (11, 12) unter Wirkung eines Innendruckes im Kultivierungsgefäß und/oder mit einem faltbaren Innenträger (21) einstellbar ist,
- die Gefäßwand (10) ist zumindest teilweise aus einem elastisch deformierbaren Material hergestellt, wobei der Abstand zwischen den Deck- und Bodenabschnitten (11, 12) unter Wirkung einer elastischen Rückstellkraft des elastisch deformierbaren Materials einstellbar ist,
- die Gefäßwand (10) weist einen Seitenabschnitt (15) aus einem elastisch deformierbaren Material auf, über den der Deckabschnitt (11) und der Bodenabschnitt (12) miteinander verbunden sind, und
- die Gefäßwand (10) weist mindestens einen Fensterabschnitt (17) auf, der für mindestens eines von einer optischen Beobachtung und einer invasiven Durchbrechung mittels eines Werkzeugs ausgelegt ist.

5. Kultivierungsgefäß gemäß einem der vorhergehenden Ansprüche, bei dem
- der Bodenabschnitt (12) zur Kultivierungsfläche (13) gegenüberliegend eine Bodenfläche mit einer Vielzahl hydrophiler Oberflächenbereiche (19) aufweist, die durch hydrophobe Oberflächenbereiche voneinander getrennt und zu den Positionen der hydrophilen Oberflächenbereiche der Kultivierungsfläche (13) passend angeordnet sind.

6. Kultivierungsgefäß gemäß einem der vorhergehenden Ansprüche, bei dem
- die Kultivierungsfläche (13) durch eine Innenfläche des Deckabschnitts (11) gebildet wird.

7. Kultivierungsgefäß gemäß einem der vorhergehenden Ansprüche, das umfasst
- mindestens eine Zwischenwand (22, 26), durch die der Innenraum (20) in mindestens zwei Horizontalkammern (23, 27, 28) unterteilt ist.

8. Kultivierungsgefäß gemäß Anspruch 7, bei dem die mindestens eine Zwischenwand (22, 26) mindestens eine der Eigenschaften aufweist
- die mindestens eine Zwischenwand (22, 26) ist aus einem Material hergestellt, das eine molekulare Diffusion erlaubt,
- die mindestens eine Zwischenwand (22, 26) weist Poren auf,
- die mindestens eine Zwischenwand (22, 26) weist Sollbruchstellen (24) auf, und
- die mindestens eine Zwischenwand ist auf einen Teilbereich des Innenraums (20) beschränkt.

9. Kultivierungsgefäß gemäß einem der Ansprüche 7 oder 8, bei dem
- die mindestens eine Zwischenwand (22, 26) ein Teil des Deckabschnitts (11) ist und die Kultivierungsfläche (13) durch eine zum Bodenabschnitt (12) weisende Oberfläche der mindestens einen Zwischenwand (22, 26) gebildet wird, oder
- die mindestens eine Zwischenwand (22, 26) ein Teil des Bodenabschnitts (12) ist.

10. Kultivierungsgefäß gemäß einem der vorhergehenden Ansprüche, das umfasst
- eine Trägereinrichtung (30), die das Kultivierungsgefäß an seiner Unterseite abstützt.

11. Kultivierungsgefäß gemäß Anspruch 10, bei dem
- die Trägereinrichtung (30) Massenelemente (31) aufweist, mit denen ein Massenschwerpunkt des Kultivierungsgefäßes im Bodenabschnitt (12) oder an diesen angrenzend gebildet wird.

12. Kultivierungsgefäß gemäß einem der vorhergehenden Ansprüche, das umfasst
- eine Medieneinrichtung (40) mit mindestens einer verschließbaren Medien-Schnittstelle (41, 43), die zur Zu- und/oder Abfuhr von flüssigen und/oder gasförmigen Medien in und aus dem Innenraum (20) eingerichtet sind.

13. Kultivierungsgefäß gemäß einem der vorhergehenden Ansprüche, das umfasst
- eine äußere Aufnahmeeinrichtung (50), die zur Aufnahme der Gefäßwand (10) eingerichtet ist und eine transparente Platte aufweist, an welcher der Deckabschnitt (11) anliegt, wenn der Arbeitsabstand zwischen dem Deckabschnitt (11) und dem Bodenabschnitt (12) eingestellt ist, und/oder
- eine äußere Klemmeinrichtung (80), mit der das Volumen des Innenraums (20) einstellbar ist.

14. Kultivierungsgefäß gemäß einem der vorhergehenden Ansprüche, bei dem
- der Deckabschnitt (11) und der Bodenabschnitt (12) linienförmige Koppelelemente (90) aufweisen, an denen der Deckabschnitt (11) und der Bodenabschnitt (12) miteinander verbunden werden können, so dass der Innenraum (20) in Vertikalkammern unterteilt wird.

15. Verfahren zur Kultivierung biologischer Zellen (1) in hängenden Tropfen (2) in einem Kultivierungsgefäß (100) gemäß einem der vorhergehenden Ansprüche, mit den Schritten
- Bereitstellung einer Suspension, welche die biologischen Zellen (1) enthält, am Bodenabschnitt (12) des Kultivierungsgefäßes,
- Benetzung der Kultivierungsfläche (13) mit der Suspension und Bildung von hängenden Tropfen (2) an der Kultivierungsfläche (13), und
- Kultivierung der biologischen Zellen (1) in den hängenden Tropfen (2).

16. Verfahren gemäß Anspruch 15, bei dem die Benetzung der Kultivierungsfläche (13) die Schritte umfasst
- Kompression des Kultivierungsgefäßes derart, dass sich der Abstand des Deckabschnitts (11) und des Bodenabschnitts (12) verringert und die Kultivierungsfläche (13) die Suspension berührt, und
- Rückstellung der Gefäßwand (10) derart, dass sich der Abstand des Deckabschnitts (11) und des Bodenabschnitts (12) vergrößert und die frei hängenden Tropfen (2) der Suspension an den Halteelementen (14) der Kultivierungsfläche (13) gebildet werden, wobei die Kompression und die Rückstellung der Gefäßwand (10) umfasst
- eine Einstellung eines Innendruckes im Kultivierungsgefäß,
- eine Betätigung eines faltbaren Innenträgers (21) im Kultivierungsgefäß, und/oder
- eine Deformation unter der Wirkung einer elastischen Rückstellkraft des Materials der Gefäßwand.

17. Verfahren gemäß Anspruch 16, mit den Schritten
- Zu- und Abfuhr von flüssigen und/oder gasförmigen Medien in und aus dem Innenraum (20).

18. Verfahren gemäß einem der Ansprüche 16 bis 17, mit mindestens einem der Schritte
- optische Beobachtung der hängenden Tropfen (2) durch mindestens einen Fensterabschnitt (17) in der Gefäßwand (10), und
- invasiven Durchbrechung mindestens eines Fensterabschnitts (17) in der Gefäßwand (10) mit einem Werkzeug und Zugriff zu dem hängenden Tropfen (2).

19. Verfahren gemäß einem der Ansprüche 16 bis 18, mit mindestens einem der Schritte
- Unterteilung des Innenraums (20) des Kultivierungsgefäßes in Horizontalkammern (23, 27, 28), und
- Unterteilung des Innenraums (20) des Kultivierungsgefäßes in Vertikalkammern (29).

## Claims

1. Culture vessel (100), which is configured for culturing biological cells (1) in hanging droplets (2), comprising
- a vessel wall (10) which has a cover section (11) and a bottom section (12), wherein
- the cover section (11) is configured for providing a culturing area (13) and the bottom section (12) is configured for receiving a liquid (3) and is arranged in an inner space of the culture vessel (100) opposite to the culturing area (13),
- the vessel wall (10) encloses an inner space (20) of the culture vessel on all sides,
- the culturing area (13) has holding elements (14) which are configured for positioning the droplets (2), wherein the culturing area (13) is able to accommodate the droplets (2) hanging freely in the inner space (20), and
- the vessel wall (10) is movable so that by means of a movement of parts of the vessel wall (10) relative to each other the holding elements (14) can be wetted with the liquid (3) from the bottom section (12),
**characterized in that**
- the vessel wall (10) is deformable and the cover section (11) and the bottom section (12) are movable relative to one another so that their mutual distance can be reduced and the holding elements (14) can be approached to the bottom section (12) .

2. Culture vessel according to claim 1, wherein the holding elements (14) comprise
- hydrophilic surface regions of the culturing area (13) which are separated from one another by hydrophobic surface regions.

3. Culture vessel according to claim 2, wherein
- the hydrophilic surface regions form a regular pattern,
- the hydrophilic surface regions are formed by a surface functionalisation of the culturing area (13), and/or
- the hydrophilic surface regions are formed by step elements on the culturing area (13).

4. Culture vessel according to one of the preceding claims, wherein the vessel wall (10) comprises at least one of the features
- the vessel wall (10) is made from a flexible film material, wherein the distance between the cover and bottom sections (11, 12) is adjustable under the effect of an internal pressure in the culture vessel and/or with a foldable inner carrier (21),
- the vessel wall (10) is made at least partially from an elastically deformable material, wherein the distance between the cover and bottom sections (11, 12) is adjustable under the effect of an elastic restoring force of the elastically deformable material,
- the vessel wall (10) has a side section (15) made of an elastically deformable material, by means of which the cover section (11) and the bottom section (12) are connected to one another, and
- the vessel wall (10) has at least one window section (17) which is configured for at least one of an optical observation and an invasive penetration by means of a tool.

5. Culture vessel according to one of the preceding claims, wherein
- the bottom section (12) has a bottom area opposite to the culturing area (13) with a plurality of hydrophilic surface regions (19) which are separated from one another by hydrophobic surface regions and are arranged matching the positions of the hydrophilic surface regions of the culturing area (13).

6. Culture vessel according to one of the preceding claims, wherein
- the culturing area (13) is formed directly by an inner surface of the cover section (11).

7. Culture vessel according to one of the preceding claims, which comprises
- at least one intermediate wall (22, 26) by means of which the inner space (20) is divided into two horizontal chambers (23, 27, 28).

8. Culture vessel according to claim 7, wherein the at least one intermediate wall (22, 26) has at least one of the properties
- the at least one intermediate wall (22, 26) is manufactured from a material which allows molecular diffusion,
- the at least one intermediate wall (22, 26) has pores,
- the at least one intermediate wall (22, 26) has predetermined breaking sites (24), and
- the at least one intermediate wall is restricted to a subregion of the inner space (20).

9. Culture vessel according to one of the claims 7 or 8, wherein
- the at least one intermediate wall (22, 26) is part of the cover section (11) and the culturing area (13) is formed by a surface of the at least one intermediate wall (22, 26) facing toward the bottom section (12), or
- the at least one intermediate wall (22, 26) is part of the bottom section (12).

10. Culture vessel according to one of the preceding claims, which comprises
- a carrier device (30) which supports the culture vessel at the underside of said culture vessel.

11. Culture vessel according to claim 10, wherein
- the carrier device (30) has mass elements (31) with which a mass centre of gravity of the culture vessel is formed in the bottom section (12) or adjacent thereto.

12. Culture vessel according to one of the preceding claims, which comprises
- a media device (40) having at least one closable media interface (41, 43) configured for the supply and/or removal of liquid and/or gaseous media into and out of the inner space (20).

13. Culture vessel according to one of the preceding claims, which comprises
- an outer receptacle device (50) for receiving the vessel wall (10) and a transparent plate against which the cover section (11) lies if the working distance between the cover section (11) and the bottom section (12) is adjusted, and/or
- an outer clamping device (80) with which the volume of the inner space (20) is adjustable.

14. Culture vessel according to one of the preceding claims, wherein
- the cover section (11) and the bottom section (12) have line-shaped coupling elements (90) at which the cover section (11) and the bottom section (12) can be connected to one another so that the inner space (20) is divided into vertical chambers (20).

15. Method for culturing biological cells (1) in hanging droplets (2) in a culture vessel (100) according to one of the preceding claims, comprising the steps
- providing a suspension, which contains the biological cells (1) at the bottom section (12) of the culture vessel,
- wetting the culturing area (13) with a suspension and forming hanging droplets (2) at the culturing area (13), and
- culturing the biological cells (1) in the hanging droplets (2) .

16. Method according to claim 15, wherein the wetting of the culturing area (13) comprises the steps
- compressing the culture vessel such that the spacing of the cover section (11) and the bottom section (12) is reduced and the culturing area (13) touches the suspension, and
- restoring the vessel wall (10) such that the spacing of the cover section (11) and the bottom section (12) is increased and the freely hanging droplets (2) of the suspension are formed on the holding elements (14) of the culturing area (13), wherein the compression and restoration of the vessel wall (10) comprises
- an adjustment of an internal pressure in the culture vessel,
- actuating a foldable inner carrier (21) in the culture vessel, and/or
- a deformation under the effect of an elastic restoring force of the material of the vessel wall.

17. Method according claim 16, with the steps
- supply and removal of liquid and/or gaseous media into and out of the inner space (20).

18. Method according to one of the claims 16 to 17, having at least one of the steps
- optical observation of the hanging droplets (2) through at least one window section (17) in the vessel wall (10), and
- invasive penetration of at least one window section (17) in the vessel wall (10) with a tool and access to the hanging droplets (2).

19. Method according to one of the claims 16 to 18, having at least one of the steps
- subdividing the inner space (20) of the culture vessel into horizontal chambers (23, 27, 28), and
- subdividing the inner space (20) of the culture vessel into vertical chambers (29).

## Revendications

1. Boîte de culture (100) qui est conçue pour la culture de cellules biologiques (1) dans des gouttes (2) suspendues comprenant
- une paroi de boîte (10) qui présente une section de couvercle (11) et une section de fond (12), dans laquelle
- la section de couvercle (11) est conçue pour la fourniture d'une surface de culture (13) et la section de fond (12) est conçue pour la réception d'un liquide (3) et est agencée dans l'espace intérieur de la boîte de culture (100) à l'opposé de la surface de culture (13),
- la paroi de boîte (10) forme de tous les côtés un espace intérieur (20) de la boîte de culture,
- la surface de culture (13) présente des éléments de retenue (14) qui sont conçus pour le positionnement des gouttes (2), dans laquelle la surface de culture (13) peut recevoir les gouttes (2) librement suspendues dans l'espace intérieur (20) et
- la paroi de boîte (10) est mobile de sorte que les éléments de retenue (14) puissent être humidifiés avec le liquide (3) de la section de fond (12) par un déplacement de parties de la paroi de boîte les unes par rapport aux autres,
**caractérisée en ce que**
- la paroi de boîte (10) est déformable et la section de couvercle (11) et la section de fond (12) sont mobiles l'une par rapport à l'autre de sorte que leur distance mutuelle puisse être réduite lors d'une déformation de la paroi de boîte (10) et les éléments de retenue (14) puissent être approchés de la section de fond (12).

2. Boîte de culture selon la revendication 1, pour laquelle les éléments de retenue (14) comprennent
- des zones de surface hydrophiles de la surface de culture (13) qui sont séparées les unes des autres par des zones de surface hydrophobes.

3. Boîte de culture selon la revendication 2, pour laquelle
- les zones de surface hydrophiles forment un motif régulier,
- les zones de surface hydrophiles sont formées par une fonctionnalisation de la surface de culture (13) et/ou
- les zones de surface hydrophiles sont formées par des éléments étagés au niveau de la surface de culture (13).

4. Boîte de culture selon l'une quelconque des revendications précédentes, pour laquelle la paroi de boîte (10) comprend au moins une des caractéristiques suivantes
- la paroi de boîte (10) est fabriquée en un matériau de feuille flexible, dans lequel la distance entre les sections de couvercle et de fond (11, 12) est réglable sous l'action d'une pression interne dans la boîte de culture et/ou avec un support interne (21) pliable,
- la paroi de boîte (10) est fabriquée au moins partiellement en un matériau élastiquement déformable, dans lequel la distance entre les sections de couvercle et de fond (11, 12) est réglable sous l'action d'une force de rappel élastique du matériau élastiquement déformable,
- la paroi de boîte (10) présente une section latérale (15) en un matériau élastiquement déformable, par le biais de laquelle la section de couvercle (11) et la section de fond (12) sont reliées l'une à l'autre et
- la paroi de boîte (10) présente au moins une section de fenêtre (17) qui est conçue pour au moins une parmi une observation optique et une interruption invasive au moyen d'un outil.

5. Boîte de culture selon l'une quelconque des revendications précédentes, pour laquelle
- la section de fond (12) présente à l'opposé de la surface de culture (13) une surface de fond avec une pluralité de zones de surface (19) hydrophiles qui sont séparées les unes des autres par des zones de surface hydrophobes et sont agencées de manière adaptée aux positions des zones de surface hydrophiles de la surface de culture (13).

6. Boîte de culture selon l'une quelconque des revendications précédentes, pour laquelle
- la surface de culture (13) est formée par une surface intérieure de la section de couvercle (11).

7. Boîte de culture selon l'une quelconque des revendications précédentes, qui comprend
- au moins une paroi intermédiaire (22, 26), par laquelle l'espace intérieur (20) est divisé en au moins deux chambres horizontales (23, 27, 28).

8. Boîte de culture selon la revendication 7, pour laquelle l'au moins une paroi intermédiaire (22, 26) présente au moins une des propriétés suivantes
- l'au moins une paroi intermédiaire (22, 26) est fabriquée en un matériau qui permet une diffusion moléculaire,
- l'au moins une paroi intermédiaire (22, 26) présente des pores,
- l'au moins une paroi intermédiaire (22, 26) présente des points destinés à la rupture (24), et
- l'au moins une paroi intermédiaire est limitée à une zone partielle de l'espace intérieur (20).

9. Boîte de culture selon l'une quelconque des revendications 7 ou 8, pour laquelle
- l'au moins une paroi intermédiaire (22, 26) fait partie de la section de couvercle (11) et la surface de culture (13) est formée par une surface tournée vers la section de fond (12) de l'au moins une paroi intermédiaire (22, 26) ou
- l'au moins une paroi intermédiaire (22, 26) fait partie de la section de fond (12).

10. Boîte de culture selon l'une quelconque des revendications précédentes, qui comprend
- un dispositif de support (30) qui appuie la boîte de culture contre son côté inférieur.

11. Boîte de culture selon la revendication 10, pour lequel
- le dispositif de support (30) présente des éléments de masse (31), avec lesquels un centre de gravité de la boîte de culture est formé dans la section de fond (12) ou de manière contiguë à celle-ci.

12. Boîte de culture selon l'une quelconque des revendications précédentes, qui comprend
- un dispositif de milieux (40) avec au moins une interface de milieux (41, 43) refermable qui est conçue pour l'alimentation et/ou l'évacuation de milieux liquides et/ou gazeux dans et hors de l'espace intérieur (20).

13. Boîte de culture selon l'une quelconque des revendications précédentes, qui comprend
- un dispositif de réception extérieur (50) qui est conçu pour la réception de la paroi de boîte (10) et présente une plaque transparente, contre laquelle la section de couvercle (11) repose, lorsque la distance de travail entre la section de couvercle (11) et la section de fond (12) est réglée et/ou
- un dispositif de serrage (80) extérieur, avec lequel le volume de l'espace intérieur (20) est réglable.

14. Boîte de culture selon l'une quelconque des revendications précédentes, pour laquelle
- la section de couvercle (11) et la section de fond (12) présentent des éléments de couplage (90) linéaires, au niveau desquels la section de couvercle (11) et la section de fond (12) peuvent être reliées l'une à l'autre de sorte que l'espace intérieur (20) soit divisé en chambres verticales.

15. Procédé de culture de cellules biologiques (1) dans des gouttes (2) suspendues dans une boîte de culture (100) selon l'une quelconque des revendications précédentes, avec les étapes suivantes :
- la fourniture d'une suspension qui contient les cellules biologiques (1), au niveau de la section de fond (12) de la boîte de culture,
- l'humidification de la surface de culture (13) avec la suspension et la formation de gouttes (2) suspendues au niveau de la surface de culture (13) et
- la culture des cellules biologiques (1) dans les gouttes (2) suspendues.

16. Procédé selon la revendication 15, pour lequel l'humidification de la surface de culture (13) comprend les étapes suivantes
- la compression de la boîte de culture de telle manière que la distance de la section de couvercle (11) et de la section de fond (12) soit réduite et la surface de culture (13) touche la suspension et
- le rappel de la paroi de boîte (10) de telle manière que la distance de la section de couvercle (11) et de la section de fond (12) augmente et les gouttes librement (2) suspendues de la suspension soient formées au niveau des éléments de retenue (14) de la surface de culture (13), dans lequel la compression et le rappel de la paroi de boîte (10) comprend
- un réglage d'une pression interne dans la boîte de culture,
- un actionnement d'un support intérieur (21) pliable dans la boîte de culture et/ou
- une déformation sous l'action d'une force de rappel élastique du matériau de la paroi de boîte.

17. Procédé selon la revendication 16, avec les étapes suivantes
- l'alimentation et l'évacuation de milieux liquides et/ou gazeux dans et hors de l'espace intérieur (20).

18. Procédé selon l'une quelconque des revendications 16 et 17, avec au moins une des étapes suivantes
- l'observation optique des gouttes (2) suspendues par au moins une section de fenêtre (17) dans la paroi de boîte (10), et
- l'interruption invasive au moins d'une section de fenêtre (17) dans la paroi de boîte (10) avec un outil et l'accès à la goutte (2) suspendue.

19. Procédé selon l'une quelconque des revendications 16 à 18, avec au moins une des étapes suivantes
- la division de l'espace intérieur (20) de la boîte de culture en chambres horizontales (23, 27, 28) et
- la division de l'espace intérieur (20) de la boîte de culture en chambres verticales (29).
